# EUROPEAN PATENT APPLICATION

(11) **EP 4 721 863 A1**
(43) Date of publication of application: **08.04.2026**
(21) Application number: 25209793.6
(22) Date of filing: 10.06.2022
(51) Int. Cl.: B01L 3/00, C08G 65/00, C08G 65/26, C08G 65/332, C08G 65/337, C08L 71/00, C08L 71/02, C09K 23/00, C12Q 1/6806

(54) **FLUOROSURFACTANTS FOR STABILIZING SINGLE- AND DOUBLE-EMULSION DROPLETS**

(30) Priority: 11.06.2021 DK PA202100621; 30.11.2021 DK BA202100110 U; 22.05.2022 DK PA202200480
(62) Divisional of application: 22741693.0
(71) Applicant: Samplix ApS, 3460 Birkerød (DK)
(72) Inventor: SKOUV, Jan, 3060 Espergærde (DK); MORGENTHALER ECHWALD, Søren, 3050 Humlebæk (DK); JUUL JACOBSEN, Mette, 3550 Slangerup (DK); JUST MIKKELSEN, Marie, 2700 Brønshøj (DK); HUSSING, Christian, 2100 København Ø (DK)
(74) Representative: Weickmann & Weickmann PartmbB

(57) **Abstract**

The invention provides a fluorosurfactant composition for use in stabilising emulsions, in particular emulsions comprising single or double-emulsion droplets; as well as improved methods for manufacture of the fluorosurfactant; wherein the surfactant comprises a perfluorocarbon chain amide covalently linked 5 to a polymer of ethylene oxide and propylene oxide. The invention also provides methods for single-cell resolution cell killing- and cell secretion-assays based on droplets stabilised by the fluorosurfactant composition.

## Description

### FIELD OF THE INVENTION

The invention provides a fluorosurfactant composition for use in stabilising emulsions, both emulsions comprising single- or double-emulsion droplets and in particularly their use for encapsulating cells. The invention also provides methods for manufacture of the fluorosurfactant compositions.

### BACKGROUND OF THE INVENTION

Emulsions comprise a discontinuous liquid phase of small droplets dispersed in a continuous liquid phase that are stabilized by a third component, typically surfactant molecules. The properties of the emulsion - mechanical, rheological, chemical - are essentially different from those of both individual liquids phases, creating complex fluids of practical interest for numerous applications.

The enormous potential of emulsion droplets initially led to their use as miniaturized reaction vessels to perform biochemical assay systems. However the real breakthrough in exploiting such reaction vessels came with advances in the droplet-based microfluidic technology as a subdomain of microfluidics. In this technology, immiscible phases are flowed through micro-channels such that homogeneous shearing of the liquids results in the formation of emulsions composed of discrete monodisperse droplets. Improvements in droplet production frequencies, ranging from a few to more than 10 kHz, and reduction in droplet volumes down to the femtolitre range; have been complimented with techniques that facilitate the manipulation, sorting, splitting, trapping or fusing of droplets in microfluidic devices.

These techniques allow for the production and precise manipulation of calibrated emulsion droplets at high rates (up to several kHz), unleashing an enormous potential for high-throughput screening applications, single cell analysis, DNA-based diagnostics or drug screening.

However, the use of emulsions as micro-reactors relies on the controlled formation, deformation and coalescence of its component droplets. This has revealed the need for superior surfactants to confer both stabilization of the droplet interface and control of the dynamics of the droplet deformations. The present invention addresses this need; by providing the surfactants needed for implementation of droplet-based bioanalysis or biodetection in all their diverse applications.

### SUMMARY OF THE INVENTION

A first aspect of the invention provides a method of synthesis of a fluorosurfactant having the formula: A-X-B or A-X-B-X-A, wherein:
each instance of A is independently F-[CF(CF₃)CF₂O]_{c}-CF(CF₃)CONH-, wherein c is greater than or equal to 30;
X, is a covalent bond; and
each instance of B is independently -[C₃H₆O]_{d}-[C₂H₄O]ₑ-[C₃H₆O]_{f}-CH₂CH(CH₃)- or - [C₃H₆O]_{g}-[C₂H₄O]ₕ-CH₃, wherein a combination of d and f is an integer greater than or equal to 2, e is an integer greater than or equal to 7, each of g and h are integers greater than 0 and a combination of g and h is an integer greater than or equal to 7;
comprising the synthesis step of reacting the carboxylic end group of a fluoropolymer having the structure F-[CF(CF₃)CF₂O]_{c}-CF(CF₃)COOH with oxalyl chloride in the presence of N,N-Dimethylformamide to obtain an activated fluoropolymer intermediate having the structure F-[CF(CF₃)CF₂O]_{c}-CF(CF₃)COCl.

A second aspect of the invention provides a composition producible by the method according to the invention, comprising:
a fluorosurfactant having the formula: A-X-B or A-X-B-X-A, wherein:
each instance of A is independently F-[CF(CF₃)CF₂O]_{c}-CF(CF₃)CONH-, wherein c is greater than or equal to 30;
X, is a covalent bond; and
each instance of B is independently -[C₃H₆O]_{d}-[C₂H₄O]ₑ-[C₃H₆O]_{f}-CH₂CH(CH₃)- or - [C₃H₆O]_{g}-[C₂H₄O]ₕ-CH₃,
   wherein a combination of d and f is an integer greater than or equal to 2, e is an integer greater than or equal to 7, and wherein each of g and h are integers greater than 0 and a combination of g and h is greater than or equal to 7, wherein the composition comprising the fluorosurfactant having the formula: AX-B or A-X-B-X-A has single-emulsion stabilization properties characterized by the formation of an emulsion comprising said composition, wherein less than 5% of single-emulsion droplets formed are coalescent droplets after at least 16 h incubation at 30°C and 10 min of incubation at 65°C at about 1 atmosphere, wherein the coalescent droplets have an average cross-sectional dimension at least 2 times the average cross-sectional dimension of the droplets formed, and wherein the size and number of the single-emulsion droplets and the coalesced droplets is determined by bright-field microscopy,
      or
   wherein the composition comprising the fluorosurfactant having the formula: AX-B or A-X-B-X-A has double-emulsion stabilization properties characterized by less than 5% of the drops viewed in microscope are coalescent, after the emulsion has been subjected to thermocycling comprising at least 40 repetitive incubations at 94°C for 3 sec followed by 60°C for 30 sec t approx. 1 atmosphere, wherein the coalescent droplets have an average cross-sectional dimension at least 2 times the average cross-sectional dimension of the droplets formed, and wherein the size and number of the single-emulsion droplets and the coalesced droplets is determined by bright-field microscopy,
      or
   wherein the composition comprising the fluorosurfactant having the formula: A-X-B-X-A, wherein:
      each instance of A is independently F-[CF(CF3)CF2O]c-CF(CF3)CONH-, wherein c is greater than or equal to 30;
      X, is a covalent bond; and
      B is -[C3H6O]d-[C2H4O]e-[C3H6O]f-CH2CH(CH3)-,
      wherein a combination of d and f is an integer greater than or equal to 2, e is an integer greater than or equal to 7, has double-emulsion stabilization properties characterized by the formation of an emulsion comprising said composition,
      wherein less than 5% of the single-emulsion drops formed are coalescent droplets after six weeks of storage at room temperature and approximately 1 atmosphere of pressure, when droplet coalescence is defined as a droplet having a diameter of at least 2 times the diameter of the average diameter of the observed double-emulsion droplets.

A third aspect of the invention provides an emulsion of single- or double-emulsion droplets comprising the composition or mixed composition of fluorosurfactants of the invention.

A fourth aspect of the invention provides a composition or mixed composition of fluorosurfactant according to the invention, for the preparation of an emulsion of single- or double-emulsion droplets.

A fifth aspect of the invention provides methods for assaying the functionality of one or more cells comprising encapsulating the one or more cells in the droplets of a water-in-fluorocarbon oil emulsion or a water-in-fluorocarbon oil-in-water emulsion, wherein said fluorocarbon oil comprises the composition or mixed composition according to the invention.

A sixth aspect of the invention provides a kit of parts for preparation of an emulsion of single- or double-emulsion droplets, comprising a composition or mixed composition of fluorosurfactant according to the invention in a first container, at least one microfluidics cartridge for forming single- and/or double-emulsion droplets, and optionally a fluorocarbon oil in a second container.

### DESCRIPTION OF THE INVENTION

### Brief description of the figures:

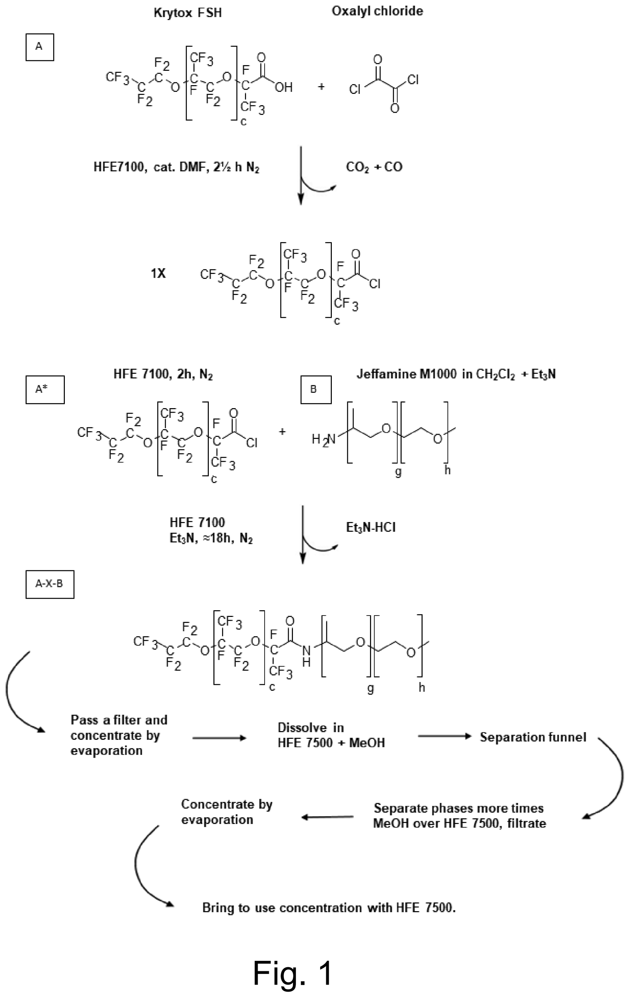

**Figure 1****:** A cartoon showing chemical synthesis of a fluorosurfactant having the formula: A-X-B also called a di-block fluorosurfactant comprising the steps of: activating the carboxylic end group of a fluoropolymer [A] by reaction with oxalyl chloride to form an activated intermediate [A*]; reacting the activated intermediate with a mono-functionalised polyether amine comprising ethylene oxide and propylene oxide monomers [B] to obtain the fluorosurfactant [A-X-B].

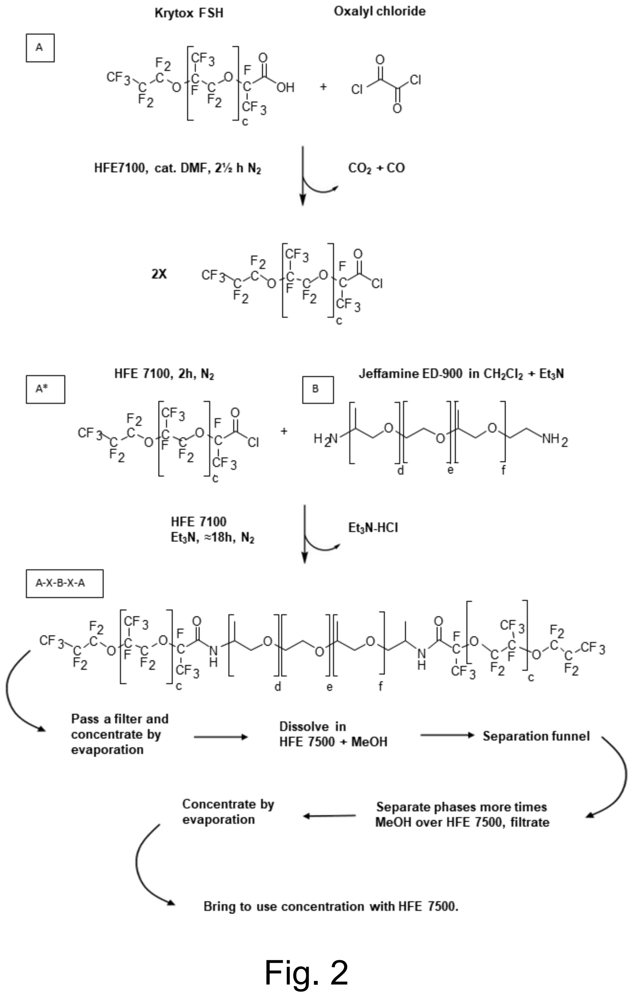

**Figure 2****:** A cartoon showing chemical synthesis of a fluorosurfactant having the formula: A-X-B-X-A also called a tri-block fluorosurfactant comprising the steps of: activating the carboxylic end group of a fluoropolymer [A] by reaction with oxalyl chloride to form an activated intermediate [A*]; reacting the activated intermediate with a di-functionalised polyether diamine comprising ethylene oxide and propylene oxide monomers [B] to obtain the fluorosurfactant [A-X-B-X-A].

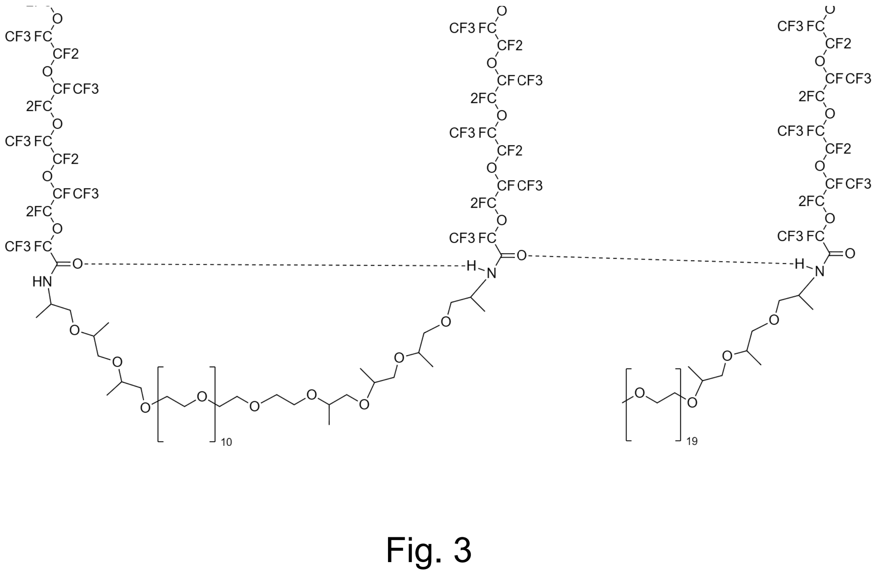

**Figure 3****:** A cartoon of a tri-block fluorosurfactant (to the left) having the formula: AX-B-X-A and a di-block fluorosurfactant (to the right) having the formula: A-X-B; showing only a portion of the fluoropolymer component [A]; wherein the polypropylene oxide monomers are predicted to form a hinge region of intermediate hydrophilicity between the more hydrophilic polyethylene oxide monomers of component [B] and the hydrophobic fluoropolymer component [A]. Intra- and inter-molecular hydrogen bonding, predicted to occur between adjacent ketone (acceptor) and amide (donor) groups located at the linkage site between the fluoropolymer (A) and the polyether head group (B), are shown by a broken line.

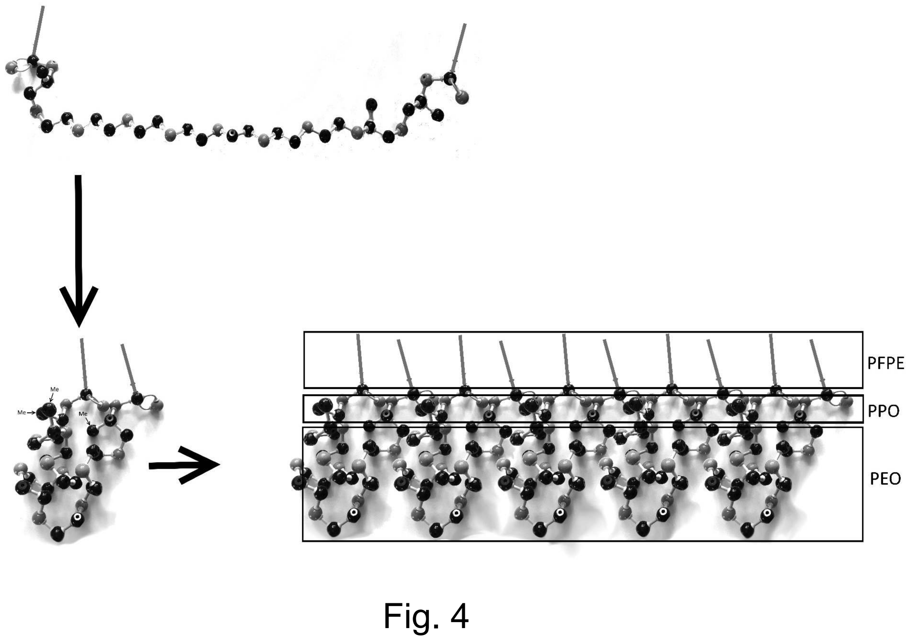

**Figure 4****:** Image of a molecular model of part of a tri-block fluorosurfactant showing: the predicted folding of the tri-block fluorosurfactant at the interface of a water: fluorocarbon oil emulsion and the possible association of surfactant molecules in three distinct "layers" at the interface, comprising a fluorophilic perfluoropolyether (PFPE) layer, an intermediate layer of polypropylene oxide (PPO), and hydrophilic layer of polyethylene oxide monomers (PEO).

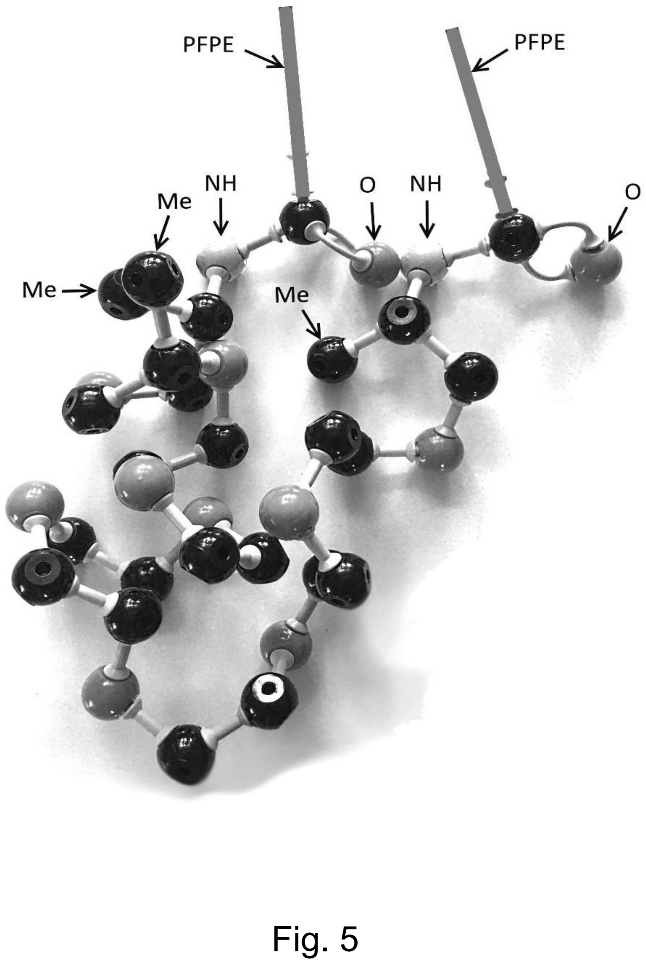

**Figure 5****:** An enlargement of the molecular model of figure 4 indicating the oxygen (O) and the nitrogen (NH), between which a hydrogen bond is predicted to be formed. Methyl groups (Me) and the perfluoropolyether (PFPE) are indicated.

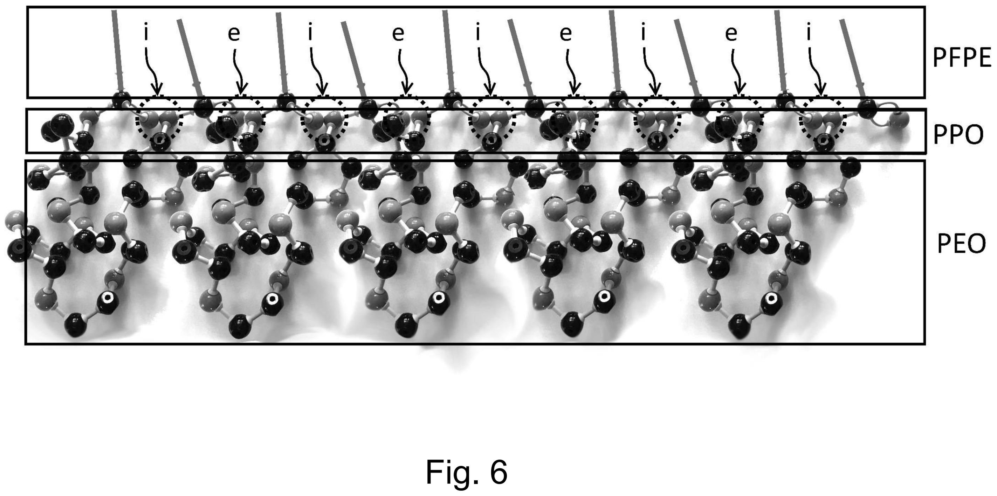

**Figure 6****:** Enlarged image of the predicted formation of both of intra- and inter-molecular hydrogen bonds (identified by broken line circles) in and between individual tri-block molecules. Intra-molecular hydrogen bonds are marked (i), and inter-molecular hydrogen bonds are marked (e). It should be noted that also di-block surfactants and mixtures of di- and tri block surfactants of the invention are predicted to be organised in a similar three-layered fashion and to form similar hydrogen bonds, see figure 3 and 4.

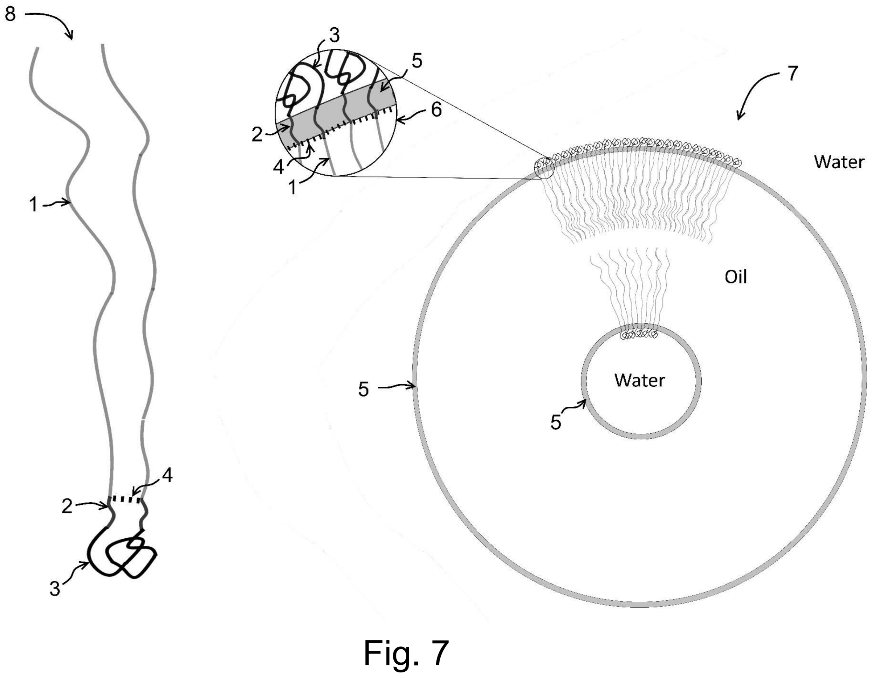

**Figure 7****:** A cartoon showing the predicted partitioning (7) of tri-block fluorosurfactant molecules at the inner and outer interfaces (5) of a double emulsion droplet formed in a water:fluorocarbon oil:water emulsion (not drawn to size). The expanded view of the outer interface (5) shows a portion of the linear perfluoropolyether chains (1) extending in parallel into the fluorocarbon oil phase (8); the polypropylene oxide hinge region (2); and the folded polyethylene oxide head groups (3) facing the aqueous phase. The inner diameter of a typical droplet is about 15-18 µm and the outer diameter is about 21-25 µm, such that the fluorocarbon-oil layer around each aqueous droplet is about 3-10 µm; sufficient to accommodate di- and/or triblock fluorosurfactants of the invention at their emulsion interfaces. The length of two opposing tri-block fluorosurfactant molecules, when aligned end-to-end, is approximately 30nm.

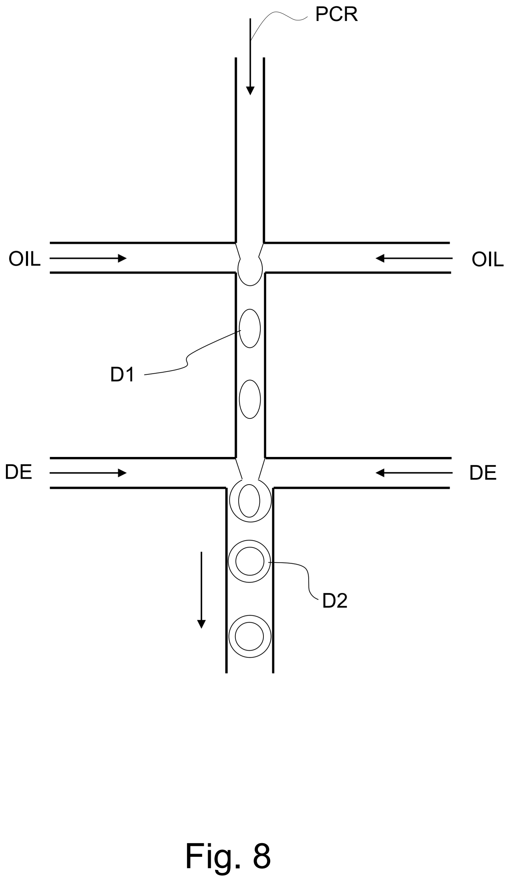

**Figure 8****:** A cartoon showing the droplet generation region of one unit of a microfluidic devise for forming double-emulsion droplets, e.g. as is the case of an Xdrop DE50 cartridge. DE is double emulsion-compatible aqueous buffer; OIL is fluorocarbon oil; and PCR is an aqueous reaction mixture including reagents for PCR amplification of nucleic acid molecules and optionally a nucleic acid sample. A corresponding microfluidic devise for forming single-emulsion droplets omits the channels for DE buffer and only has a single junction formed by oil and PCR reagent channels connected to an output channel. D1 is a single emulsion droplet, D2 is a double emulsion droplet. Arrows indicate flow directions.

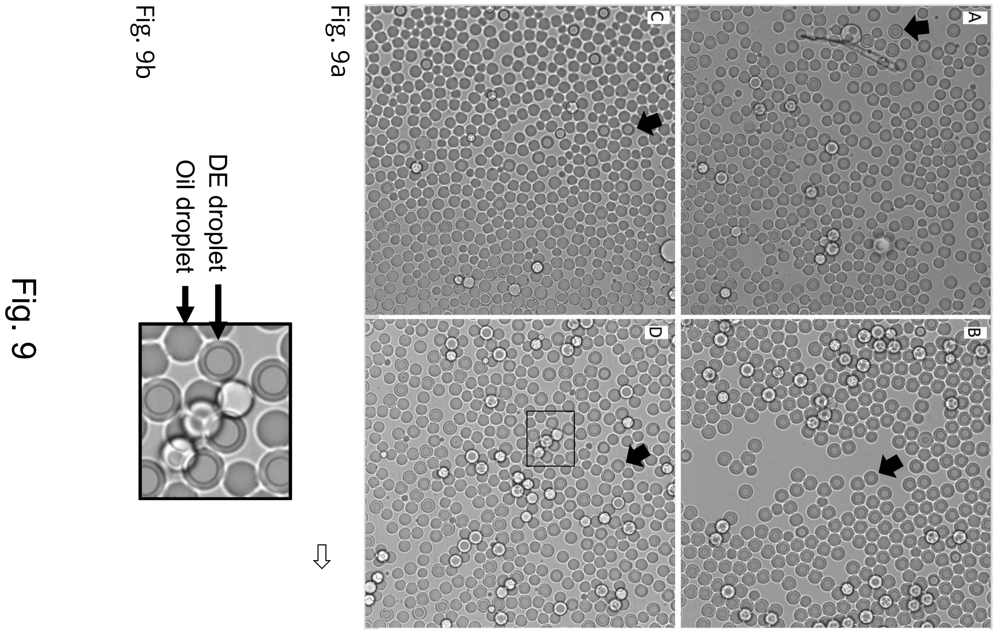

**Figure 9** **A:** Bright-field image of double emulsion droplets produced with 1 % fluorosurfactant (FS). The FS used is triblock fluorosurfactant alone (A), di-block fluorosurfactant alone (B), a 9:1 triblock/diblock mixture (C) and an 8:2 triblock/diblock mixture (D). The arrows indicate an example of a double emulsion droplet. A number of oil drops also appear.
**Figure 9** **B:** An enlargement of the square indicated in fig. 9 panel D. Arrows point to a double emulsion droplet and an oil droplet.
**Figure 10****:** Bright-field image of single emulsion droplets after incubation for 16h at 30°C followed by 10 minutes of incubation at 65°C. The ratio tri-block:di-block fluorosurfactant varies. Tri-block to di-block ratios were A) 5%:0%; B) 4,5%:0,5%; C) 4%:1%; D) 3,5%:1,5%; E) 3,0%:2% and F) 2,5%:2,5% w/v. Each emulsion shown comprised a final concentration of 5% w/v fluorosurfactant. Part of the counting-grid of the Bürker-Türk counting chamber is seen in images A and B. Large square in grid is 0,2x0,2 mm.
**Figure 11****.** Shows one embodiment of the split well insert that fits into a sample supply well of an Xdrop^{™} droplet generating cassette, e.g. item number CA10100, Samplix ApS, Birkerød, Denmark.
   FIG. 11A shows in section an example where an insert is placed in the left sample supply well, (well C 1), but not in the next sample feed well (well C 2).
   FIG. 11B is a 3D line drawing of a preferred embodiment of the insert.
   FIG. 11C is a section of 2B flush with the partition between chambers 1 and 2.
   FIG. 11D shows section E enlarged.
   "h" indicates the height of the insert.
**Figure 12****.** Illustrate the Xdrop DE50 cartridge. The cartridge is designed to fit into a specially adapted instrument (the Xdrop instrument, cat. no. IN00100, Samplix ApS, Birkerød, Danmark) . An embodiment of the instrument is shown in Figure 12. When inserted into the Xdrop instrument and run following the recommendations of the manufacturers, the cartridge produces stable double emulsion droplets having a diameter of approximately 50 µm.
   FIG. 1A is a 3D line drawing. FIG. 1B shows the cassette seen from the lower left side. FIG. 1C is a section along A-A.
   The droplet forming microfluidics of the cartridge i shown i figure 8.

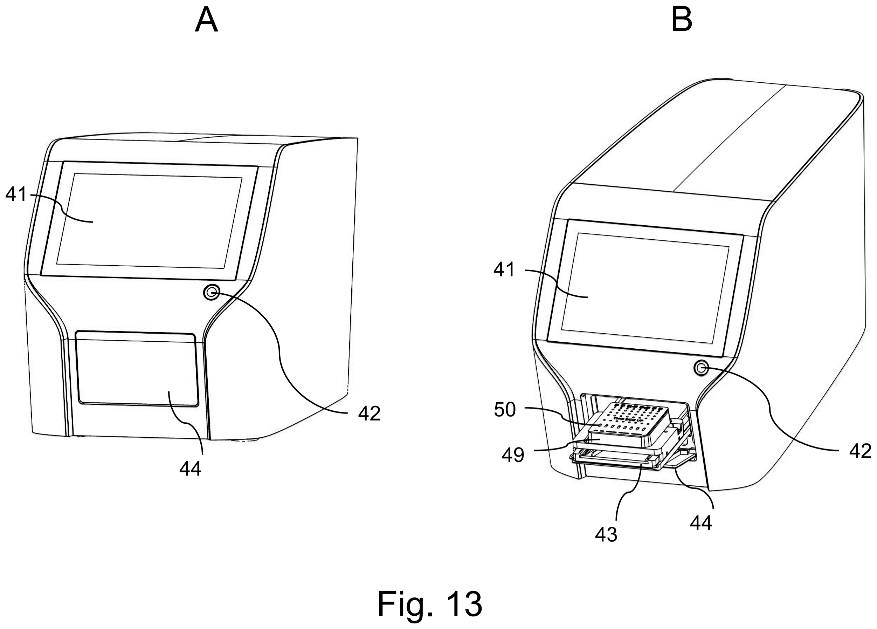

**Figure 13****.** Illustrate the Xdrop instrument, (cat. no. IN00100, Samplix ApS, Birkerød, Danmark) . A open position of the drawer of the instrument, B closed position.

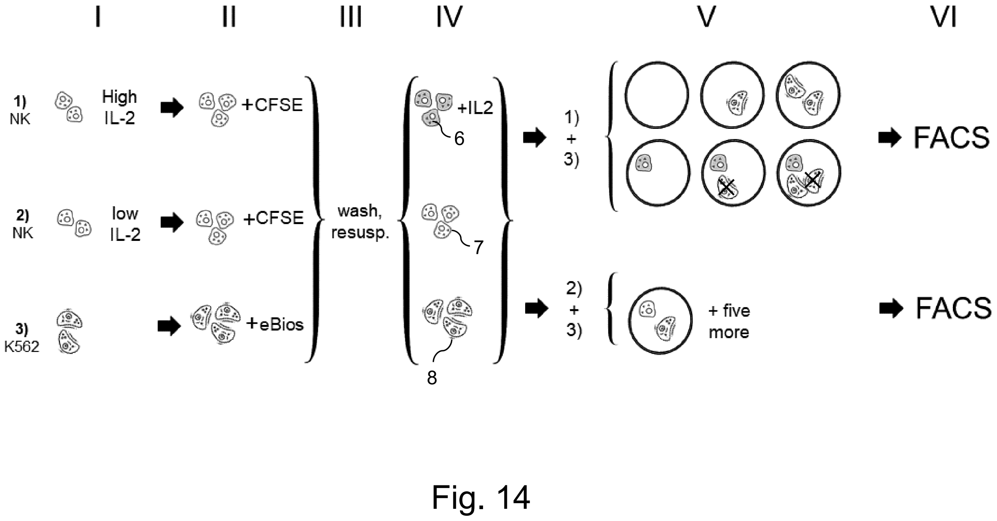

**Figure 14****.** Illustrating the steps of the NK-92/K562 killing assay of example 9.
   The steps of the assay are:
   I: growth and preactivation of cells,
   II: differential staining of the two cell-types,
   III: washing and resuspending cells in medium,
   IV: activate effector NK cells,
   V: droplet production, and
   VI: incubation and analysis by a flow-cytometer or a FACS.

   "CFSE "is CellTrace^{™} CFSE dye (Cat. no. C34554, Invitrogen, MA, USA) eBioscience^{™} Cell Proliferation Dye eFluor^{™} 670 (Cat. no. 65-0840-85, Invitrogen, MA, USA) "eBios" is eBioscience^{™} Cell Proliferation Dye eFluor^{™} 670 (Cat. no. 65-0840-85, Invitrogen, MA, USA). 6 indicate an IL-2 activated natural killer (NK) cell, 7 a non-activated NK cell, and 8 a K562 target cell.
   [6] designate a IL-2 activated natural killer (NK) cell, [7] NK cell, and [8] a K562 target cell.
   "×" indicate a killed K562 target cell.

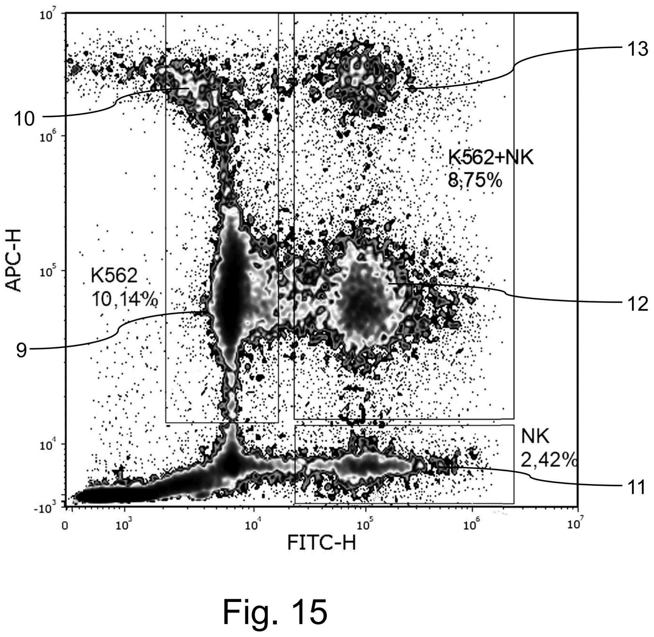

**Figure 15****.** illustrating a typical outcome of the FACS analysis of a NK-92/K562 killing assay of example 9 when plotting the green (FITC) signal vs. the red (APC) signal. populations corresponding to droplets with one K562 cell [9], two K562 cells [10], one NK-92 cell [11], one NK-92 cell and one K562 cell [12], and one NK-92 cell and two K562 cells [13] is seen.
**Figure 16****.**
   Functional cell killing obtained with single-cell resolution.

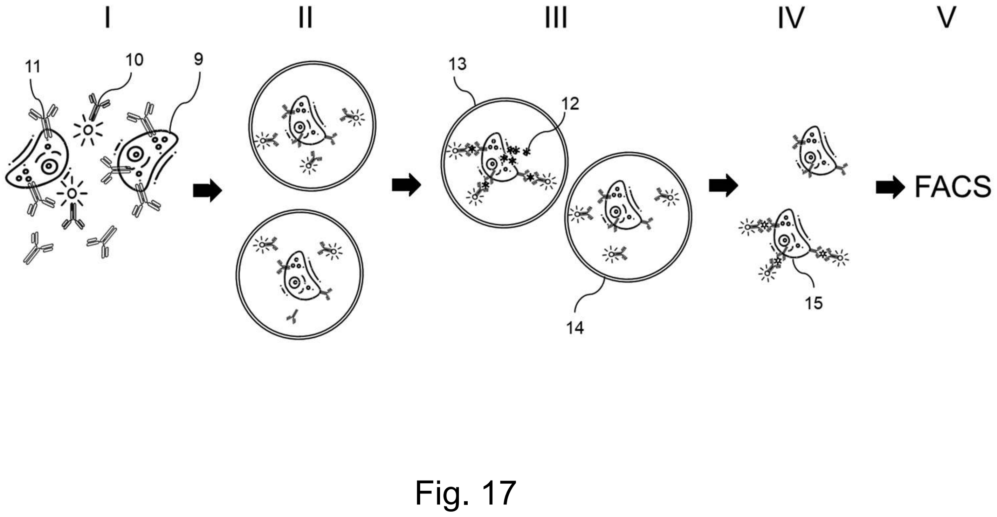

**Figure 17****.** Illustrating the steps of the single-cell protein secreation assay of example 10.
   The steps of the assay are:
   I: Mix cells with an assay containing
      a. A reagent that binds to the surface of the cells and to an interleukin
      b. An antibody that binds to the same interleukin and which is coupled to a fluorescent molecule.
   II: Produce double-emulsion droplets containing cells and assay-components
   III: Incubate double-emulsion droplets in a CO₂ incubator
   IV: Break the droplets to release the cells, and
   V: Analyze cells on a flow-cytometer or a FACS. Only cells secreting the specific interleukin will bind the fluorescently labelled antibody on the surface of the cells and can be identified as such by flow-cytometry.

   [9] Producer cell (NK-92 cells or Human Peripheral Blood Mononuclear Cells (PBMCs))
   [10] Interleukin-binding, fluorophore labelled antibody
   [11] Antibody binding to both cell surface and an interleukin
   [12] interleukin (IFN y or TNF α)
   [13] Interleukin-producing cell
   [14] cell not producing interleukin
   [15] only interleukin-producing cells will fluoresce.

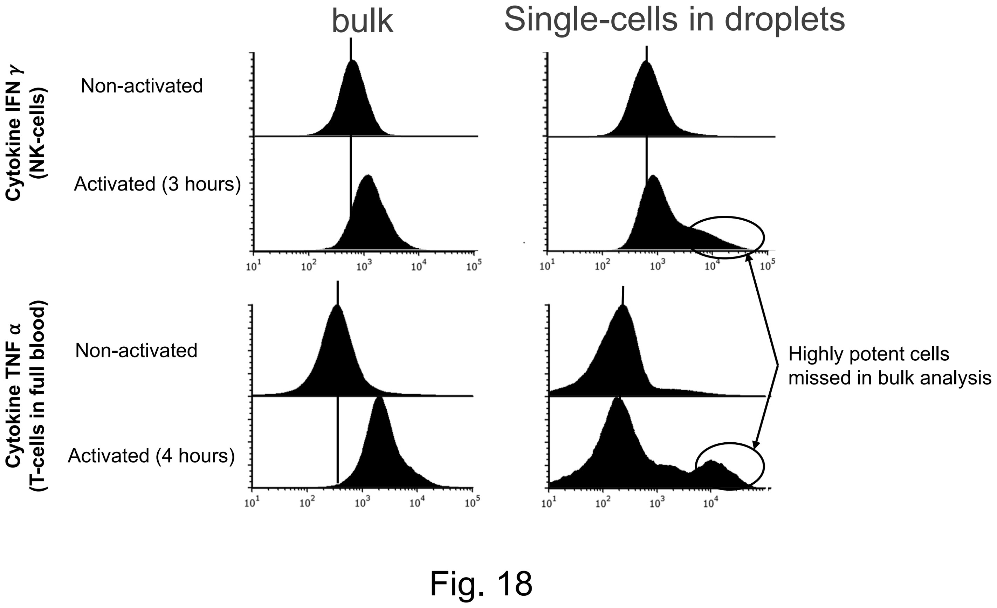

**Figure 18****.** Flow-cytometric analysis of activated cells recovered left: bulk experiment right: single cells encapsulated in double-emulsion droplets. Single-cell resolution reveals a functional heterogeneity of cell populations (example 10).

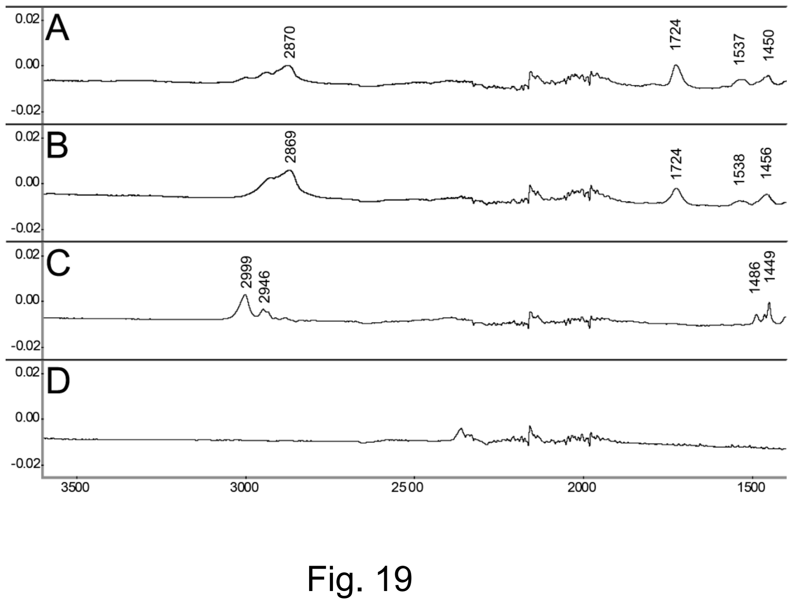

**Figure 19****.** ATR-FTIR spectra showing the region between 3600 cm-1 and 1400 cm-1. Peak values are indicated. A is the FTIR spectrum of the tri-block fluorosurfactant (82.5 % w/w in Novec 7500), B is the FTIR spectrum of the di-block fluorosurfactant (96 % w/w in Novec 7500), C is the FTIR spectrum of the Novec 7500 solvent, and D is the background signal of the instrument.

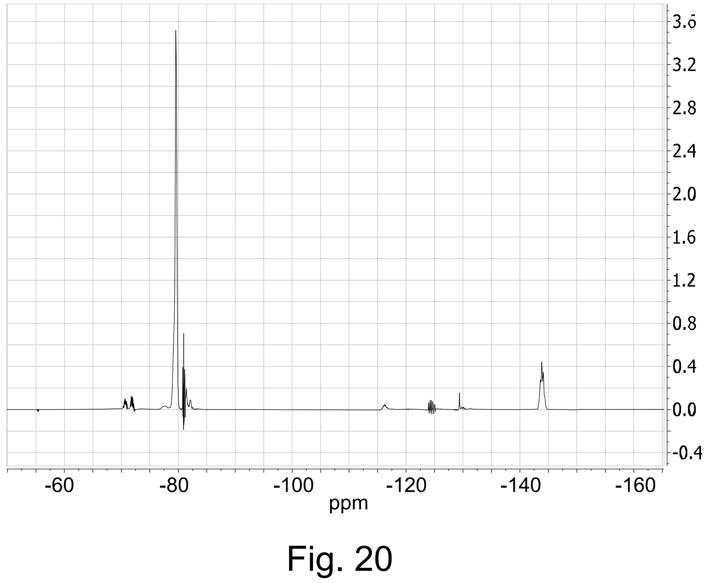

**Figure 20****.** ¹⁹F Nuclear Magnetic Resonance Subtraction spectrum (Novec 7500 signal subtracted from the tri-block in Novec 7500 signal) to highlight the tri-block signals.

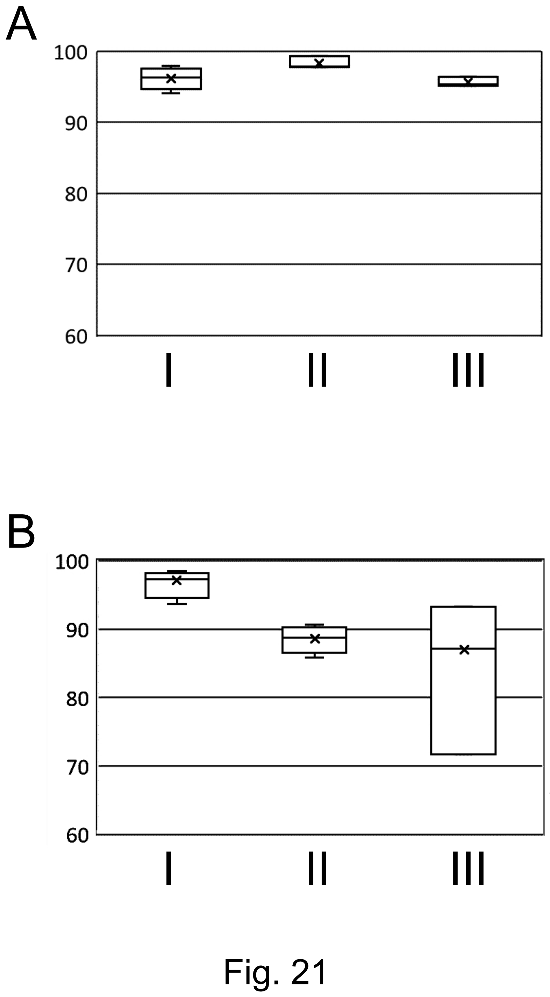

**Figure 21A****.** Viability-% of HEK cells after 24 hours incubation in growth medium with either no oil (I), 37 % v/v oil (5% w/w surfactant in Novec 7500) of the tri-block fluorosurfactant (II), or the di-block fluorosurfactant (III). The box marks the 25 % to the 75 % quartile, line inside box the median, and X average.
**Figure 21B****.** Viability-% of Ramos cells after 24 hours incubation in growth medium with either no oil (I), 36 % v/v oil (5% w/w surfactant in Novec 7500) of the tri-block fluorosurfactant (II), or the di-block fluorosurfactant (III). The box marks the 25 % to the 75 % quartile, line inside box the median, and X average.

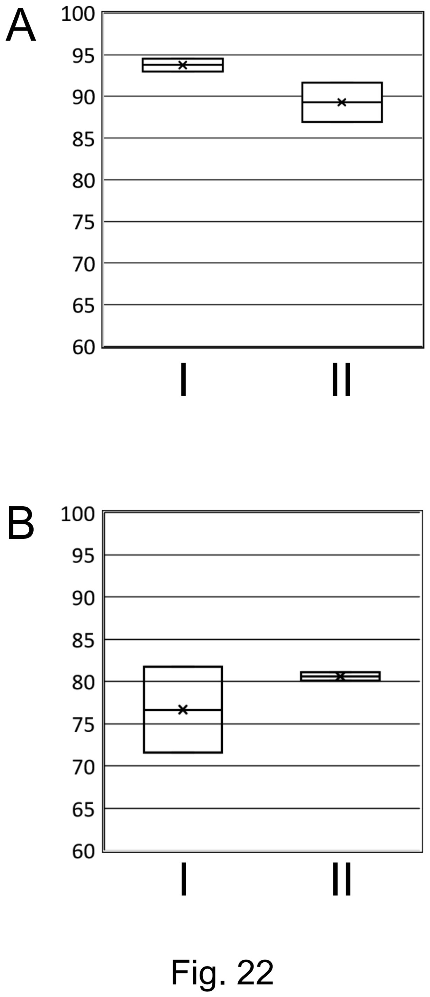

**Figure 22A****.** Viability-% of Ramos cells encapsulated in double-emulsion droplets with a diameter of approximately 50 µm (DE50 droplets) and suspended i growth medium. I, viability after 3 hours of incubation at 37 °C and 5 % CO₂ II, viability after 22 hours of incubation at 37 ⁰C and 5 % CO₂. The box marks the 25 % to the 75 % quartile, line inside box the median, and X average.
**Figure 22B****.** Viability-% of NK cells encapsulated in double-emulsion droplets with a diameter of approximately 50 µm (DE50 droplets) and suspended i growth medium. Viability after 23 hours of incubation at 37 °C and 5 % CO₂ encapsulated in droplets stabilised by the tri-block fluorosurfactant, I, or stabilised by the di-block fluorosurfactant, II. The box marks the 25 % to the 75 % quartile, line inside box the median, and X average.

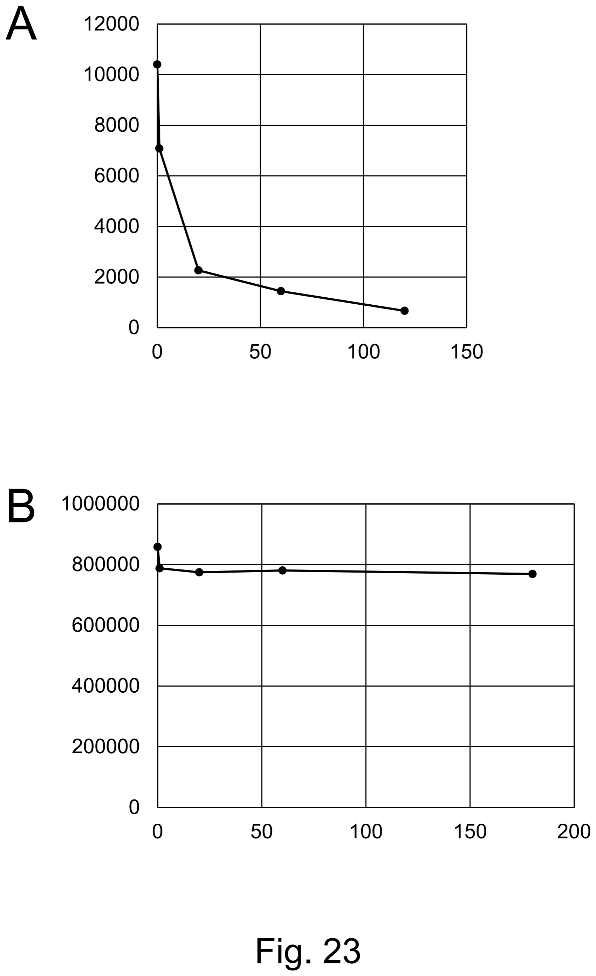

**Figure 23A****.** Decay (in minutes) of the resorufin fluorescence signal from resorufin-comprising droplets stabilized with the tri-block fluorosurfactant. Initially the resorufin concentration in the aqueous phase was 5 µM.
**Figure 23B****.** Decay (in minutes) of the fluorescein fluorescence signal from fluorescein-comprising droplets stabilized with the tri-block fluorosurfactant. Initially the fluorescein concentration in the aqueous phase was 5 µM.

### Abbreviations:

**CAR-T:** Chimeric antigen receptor T cells.
**DMF:** is N,N-Dimethylformamide
**DCM:** is dichloromethane (CH₂Cl₂)
**Et3N:** is triethyamine (C₂H₅)3N).
**FACS:** Fluorescence-activated cell sorting or the instrument used to perform fluorescence-activated cell sorting.
**FSH:** Commercial name of perfluorinated polyether used as hydrophobic block in fluorinated surfactants.
**HFE-7100:** is a hydrofluoroether (HFE) solvent, based on a mixture of 1,1,2,3,3,3-hexafluoro-1-methoxy-2-(trifluoromethyl)propan and 1,1,2,2,3,3,4,4,4-nonafluoro-1-methoxybutan (also referred to as Novec^{™}7100 and supplied by 3M^{™} A/S, Copenhagen S, Denmark).
**HFE-7500:** is 3-Ethoxyperfluoro (2-methylhexan) known as Novec^{™} 7500 Engineered fluid Cas. no. 297730-93-9 and supplied by 3M^{™} A/S, Copenhagen S, Denmark, also referred to as a fluorinated oil.
**MDA:** is Multiple Displacement Amplification (MDA) of a DNA molecule mediated by an enzyme, preferentially Φ29 DNA polymerase, in the presence of random hexamer primers that anneal to the DNA molecule. Compared with conventional PCR amplification techniques, MDA does not employ sequence-specific primers but amplifies all DNA, generating larger sized products with a lower error frequency, and works at a constant temperature (circa 30 °C).
**NK cell:** Natural killer cells.
**PCR:** is Polymerase Chain Reaction leading to the amplification of a nucleic acid molecule and mediated by a DNA polymerase enzyme in the presence of dNTPs and one or more DNA primer that can anneal to the nucleic acid molecule and be extended by the polymerase.
**RT:** room temperature, preferably in the range of 20 - 30 °C

### Detailed description of the invention:

**Fluorosurfactants,** as described herein, are surfactants that serve to stabilize emulsions derived from two or more immiscible liquids, where the surfactants partition to the interface between the immiscible liquids in the emulsion. In particular, fluorosurfactants find use in the stabilization of aqueous droplets dispersed in a fluorophilic continuous phase (e.g. single-emulsion droplets), as well as aqueous droplets, each encapsulated within a larger droplet of fluorophilic liquid, that are dispersed in a bulk aqueous continuous phase (e.g. double-emulsion droplets). Structurally, the fluorosurfactants are comprised of a fluorophilic tail that is soluble in a fluorophilic (e.g., fluorocarbon) continuous or discontinuous phase, and a headgroup that is soluble in an aqueous phase. The headgroup and the tail are covalently linked via a linking moiety. This chemical structure, of a fluorophilic tail linked to a headgroup, provides a suitable geometry for forming stabilized single- and double-emulsion droplets where an aqueous phase is dispersed in a continuous fluorophilic or continuous aqueous phase, respectively. The chemical composition of the headgroup prevents or limits the adsorption of molecules at the interface between the one or more surfactants and the discontinuous aqueous phase. This configuration allows the droplet to serve, for example, as a secluded reaction space for certain chemical and/or biological reactions. Advantageously, fluorosurfactant compositions or mixed fluorosurfactant compositions of the invention provide sufficient stabilization against coalescence of droplets, without interfering with chemical processes or reactions carried out inside the droplets.

The fluorosurfactants can have one or more main fluorophilic chains (A) soluble in the fluorophilic phase of an emulsion, where one end of each of the one or more A chains is linked to a chain (B) that is not soluble in the fluorophilic phase of the emulsion (e.g., the B chains that are soluble in the aqueous phase). The fluorosurfactants may have the structure of a di-block (e.g., A-X-B) or a tri-block, where one component of the fluorosurfactant (i.e. "A") is soluble in the fluorophilic phase and another component of the fluorosurfactant (e.g., "B") is soluble in the aqueous phase; and where "X" represents a linking moiety between the headgroup (B) and the tail (A).

**The one or more "fluorophilic" chains** (A) in the surfactant is an approximately linear fluorinated hydrocarbon, that is perfluorinated, i.e., wherein all of the hydrogen atoms of the carbon chain are replaced by fluorine atoms. Fluorophilic surfactants compatible with the present invention have low toxicity, low surface tension, and the ability to dissolve and transport gases.

**The headgroup** (B) of the fluorosurfactant can be described as having essentially two incompatible roles to play. On the one hand the headgroup must provide a biologically inert but hydrophilic surface that extends from the interface of the emulsion into an aqueous phase. On the other hand the headgroup must allow the fluorophilic chains, to which it is attached, to align and allow the packing of the surfactant molecules in an emulsion to form a stable interface. Providing a suitable headgroup is particularly challenging due to the fact that perfluoridated carbon chains of a fluorosurfactant are known to be among the most non-polar compounds known. The present invention is the first to recognize and address this problem by providing a headgroup that meets this need, and whose unique structure is detailed below.

**An emulsion** is a dispersion of small droplets in a continuous phase, stabilized by a third compound, typically comprising a surfactant. Since immiscible liquids tend to separate into two distinct phases, the presence of the "surfactant" serves to reduce surface tension between the at least two immiscible liquids and/or to stabilize the interface. An emulsion, as described herein, includes a discontinuous or disperse phase (i.e., the isolated phase stabilized by a surfactant) formed of an aqueous substance. The continuous phase may be formed of a fluorophilic substance (e.g., a fluorocarbon oil). In some embodiments, the present invention provides water-in-fluorocarbon oil emulsions having an aqueous phase dispersed in a fluorocarbon continuous phase. Such an emulsion may also be referred to as a "single-emulsion" which comprise "single-emulsion droplets". In other embodiments, the invention provides water-in-fluorocarbon oil-in-water emulsions having an aqueous phase encapsulated in a fluorocarbon oil phase and dispersed in an aqueous continuous phase. Such an emulsion may also be referred to as a "double-emulsion" which comprise "double-emulsion droplets".

**Droplets** are discontinuous single-emulsion aqueous droplets dispersed in a fluorocarbon oil phase or double-emulsion droplets (aqueous phase droplets encapsulated in a fluorocarbon phase) dispersed in an aqueous phase. Typically said single- or double- emulsion droplets have an average cross-sectional dimension (or diameter) of greater than 25 nm. In some embodiments, the average cross-sectional dimension of the droplets is greater than 50 nm, greater than 100 nm, greater than 250 nm, greater than 500 nm, greater than 1 micron, greater than 5 microns, greater than 10 microns, greater than 50 microns, greater than 100 microns, or even greater than 200 microns. As used herein, the average cross-sectional dimension of a droplet is the diameter of a perfect sphere having the same volume as the droplet.

Embodiments of the invention are described below:

### I. A method of synthesis of a fluorosurfactant of the invention

According to a first aspect, the present invention provides a method for the synthesis of a fluorosurfactant having the formula: A-X-B or A-X-B-X-A, wherein:
each instance of A is independently F-[CF(CF₃)CF₂O]_{c}-CF(CF₃)CONH-, wherein c is greater than or equal to 30; X, is a covalent bond; and
each instance of B is independently -[C₃H₆O]_{d}-C₂H₄O]ₑ-[C₃H₆O]_{f}-CH₂CH(CH₃)- or - [C₃H₆O]_{g}-[C₂H₄O]ₕ-CH₃, wherein a combination of d and f is an integer greater than or equal to 2, e is an integer greater than or equal to 7, each of g and h are integers greater than 0 and a combination of g and h is an integer greater than or equal to 7; where the method comprises the synthesis step of reacting the carboxylic end group of a fluoropolymer having the structure F-[CF(CF₃)CF₂O]_{c}-CF(CF₃)COOH with oxalyl chloride ((COCl)₂) in the presence of N,N-Dimethylformamide (DMF) to obtain an activated fluoropolymer intermediate having the structure F-[CF(CF₃)CF₂O]_{c}-CF(CF₃)COCl.

The use of oxalyl chloride to activate the carboxylic end group of the fluoropolymer, as shown below, has a number of important advantages over alternative methods for activating a fluoropolymer substrate.

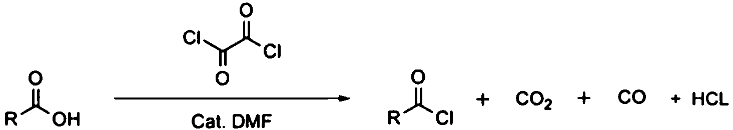

Firstly the oxalyl chloride and other bi-products of the reaction are easily separated from the activated product, for example the bi-products CO₂, CO and HCI can be removed by evaporation under reduced pressure. Secondly, the reaction with (COCl)₂ in DMF is milder and more selective for the carboxylic end group of the fluoropolymer.

Alternative methods, such as using thionylchloride (SOCI₂), to a lesser degree provide such advantages, in particular because the bi-products of such reaction (e.g. the hydration products of SO₂) are difficult to remove by simple evaporation and the reaction is less specific.

The activated fluoropolymer is then covalently linked to a polar polyether head group (B); where the head group is preferably either a mono- or a di-functionalized polyetheramine, more preferably a mono- or di-functionalized amine polymer of ethylene oxide (EO) and propylene oxide (PO) having the structure:

NH₂-[C₃H₆O]_{g}-[C₂H₄O]ₕ-CH₃ or NH₂-[C₃H₆O]_{d}-[C₂H₄O]ₑ-[C₃H₆O]_{f}-CH₂CH(CH₃)-NH₂ .

Thereby less hydrophilic propyl groups will be positioned between the highly hydrophobic perfluorated section and the hydrophilic polyethylene oxide section. Since the propylene monomers in the fluorosurfactant molecule are predicted to partition to and span the droplet interface this allows the highly polar perfluorated chain to remain in the fluorocarbon oil phase, enhancing the emulsion stabilizing effect of the fluorosurfactant.

The product of the method of the invention may comprise a di-block fluorosurfactant having the formula: A-X-B (see figure 1), whose linear chemical structure is:

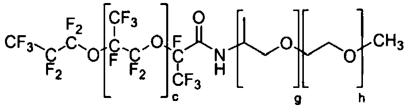

The product of the method of invention may comprise a tri-block fluorosurfactant having the formula: A-X-B-X-A (see figure 2), whose linear chemical structure is:

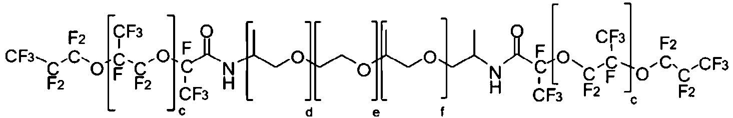

The length of the fluoropolymer is defined by "c" which is preferably an integer between 30 and 50; more preferably between 35 and 48; even more preferably 39 and 44; this being compatible with both single and double-emulsion droplets, where in the latter case the dimensions of the fluorocarbon oil phase might be limited by the dimensions of each oil droplet encompassing each aqueous droplet.

When the polar head-group is a mono-functionalized amine polymer having the structure: NH₂-[C₃H₆O]_{g}-[C₂H₄O]ₕ-CH₃, then preferably g is an integer selected from the group: 1, 2, 3, 4 and 5; h is an integer of 6 to 20, and the combination of g and h is between 7 and 25.

When the polar head-group is a di-functionalized amine polymer having the structure: NH₂-[C₃H₆O]_{d}-[C₂H₄O]ₑ-[C₃H₆O]_{f}-CH₂CH(CH₃)-NH₂, then preferably the combination of d and f is an integer between 2 and 8, preferably between 3 and 7, e is an integer between 7 and 20, preferably between 9 and 14,.

The fluoropolymer (A) herein, is highly non-polar and fluorophilic, such that when linked to the polar polyether head group (B) of the fluorosurfactant, it will assume an approximately linear conformation in the fluorocarbon phase of an aqueous-oil (or aqueous-oil-aqueous) emulsion. The polar headgroup (B), to which it (A) is linked, faces the aqueous phase of the emulsion. The efficacy of the surfactant relies on the ability of the polar head groups to pack together at the emulsion interface, such as to provide a water-soluble, largely continuous, inert and non-absorptive molecular lining on the inner surfaces (and outer surfaces) of the single- and double-emulsion droplets. While not wishing to be bound by theory, it is credible that the chemical structure of the amide linkage between the fluoropolymer (A) and the polyether head group (B), together with the terminal propyl groups of the polyether head group (B) play a key role in conferring the fluorosurfactant compositions of the invention with improved single- and double-emulsion stabilizing properties as compared to those in the art.

More specifically, intra- and inter-molecular hydrogen bonding is predicted to occur between adjacent ketone (acceptor) and amide (donor) groups located at the linkage site between the fluoropolymer (A) and the polyether head group (B). This interaction is predicted to tether individual and adjacent di- and tri-block molecules together (see figures 3, 4, 5 and 6). 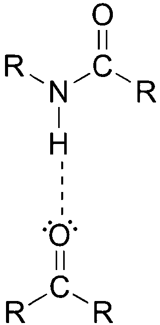
*hydrogen bond between a*
*ketone (acceptor) and an*
*amide (donor).*
*(from Organic Chemistry 1:*
*An open textbook,*
*https:*//*courses.lumenlearning.com*/*)*

Additionally, the presence of terminal propyl groups in the polyether head group (B) that are less hydrophilic, are predicted to allow these terminal groups to form a hinge region that can span the emulsion interface so as to limit or avoid aqueous phase exposure of the highly non-polar fluoropolymer (A) to which the head group is linked - thereby enhancing the stability of the emulsion (figure 4, 5, 6 and 7).

The improved properties of single- and double-emulsion droplets employing the fluorosurfactant of the invention are demonstrated in Examples 3 to 8.

### II. Fluorosurfactant composition of the invention

The method of synthesis of a fluorosurfactant (species of fluorosurfactant) of the invention, as described above (I), yields a composition enriched in the fluorosurfactant having the formula: A-X-B or A-X-B-X-A. The single-emulsion stabilization properties of the composition are characterized by the formation of an emulsion comprising said composition wherein less than 5% of droplets formed are coalescent droplets after at least 16 hours incubation at 30°C followed by 10 min of incubation at 65°C at about 1 atmosphere, wherein the coalescent droplets have an average cross-sectional dimension at least 2 times the average cross-sectional dimension of the droplets formed, and wherein the size and number of the single-emulsion droplets and the coalesced droplets is determined by bright-field microscopy.

The stability of double-emulsion droplets are assayed by performing a PCR (40 cycles) and FACS as illustrated in Example 4.

Example 11 emphasize the extraordinary stability of the single-emulsion stabilization properties of the compositions of the invention. Even after six weeks storage at room temperature and approximately 1 atmosphere of pressure less than 5% of the drops viewed in microscope were coalescent. Droplet coalescence was defined as a droplet having a diameter of at least 2 times the diameter of the average diameter of the observed double-emulsion droplets.

The chemical structure of the species of fluorosurfactant product, as described herein, resulting from the described method and respective chemical reaction schemes, can be determined by analytical tools well known in the art (more specifically IR and NMR spectroscopy).

Tools for the further purification of the fluorosurfactant (species of fluorosurfactant) include further rounds of phase separation. However the composition directly produced by the described method of the invention, confers all of the described advantageous emulsion stabilizing properties and droplet stability without the need to further enrich for the component fluorosurfactants. Indeed, it is deemed plausible that other minor but unidentified components (e.g substrates or intermediates) present in the composition contribute to the observed properties of the fluorosurfactant composition produced. In a preferred embodiment, the composition is produced by the method set out in item 8; and reduced to practice in Examples 1 and 2.

The composition produced by the method of the invention is either enriched for the fluorosurfactant having the formula: A-X-B or enriched the fluorosurfactant having the formula: A-X-B-X-A. Compositions produced by the method, for example a first composition enriched with a fluorosurfactant having the formula: A-X-B (di-block fluorosurfactant) and a second composition enriched in a fluorosurfactant having the formula: A-X-B-X-A (tri-block fluorosurfactant); may be combined in almost any desired ratio. Examples 3 to 8 demonstrate that stable emulsions are obtained both using a 1% up to a 5% di-block or tri-block fluorosurfactant composition and also using a wide range of mixed tri-block and di-block fluorosurfactant compositions. Stable emulsions were obtained using compositions comprising di-block and/or tri-block fluorosurfactants in a tri-block:di-block ratio of 10:0; 9:1; 8:2; 7:3; 6:4; 5:5 and 0:10.

### III. Methods for identification of fluorosurfactants having the formula: A-X-B or the formula: A-X-B-X-A

Fluorosurfactants, such as those in the composition of the invention, can be characterized by infrared spectroscopy (to identify key bonds), size exclusion chromatography (to determine molecular weight); and in particularly by ¹⁹F-, ¹³C- and ¹H-NMR spectroscopy (to determine the molecular structure). A ¹⁹F-NMR spectrum will generate characteristic signals corresponding to a perfluoropoly(propylene oxide) linked to a PEG through an amide bond. A ¹³C-NMR spectrum provides further aspects of their structure, in particularly the number of repeating subunits in each molecule, while similar structural details can also be obtained from a ¹H-NMR spectrum.

Both the di-block (formula: A-X-B) and the tri-block fluorosurfactant (formula: A-X-B-X-A) were analysed by fourier transform infrared (FTIR) spectroscopy. In addition the tri-block fluorosurfactant was analysed by ¹⁹F Nuclear Magnetic Resonance. See example 12. The analysis supported the anticipated structure of the fluorosurfactants shown previously.

### IV. Emulsions comprising the fluorosurfactant composition of the invention

The invention further provides an emulsion composed of:
i. aqueous droplets dispersed in an oil continuous phase or
ii. oil-encapsulated aqueous droplets dispersed in an aqueous continuous phase,
wherein said emulsion comprises a composition or mixed composition of fluorosurfactant and the oil is a fluorocarbon. The stability of droplets in both single- and double-emulsions formed using the composition of the invention, are those defined above in section II.

The fluorosurfactant composition included in the emulsion may be enriched with either a di-block fluorosurfactant; or a tri-block fluorosurfactant; or the fluorosurfactant composition may be provided as a mixture of compositions enriched respectively with di-block fluorosurfactant; and a tri-block fluorosurfactant, in any desired ratio. In one embodiment, emulsions are prepared using a 1 to 10% solution, more preferably about a 3 to 5% solution, of the tri-block fluorosurfactant composition (without any di-block fluorosurfactant), since this was found to confer surprising degree of emulsion stability.

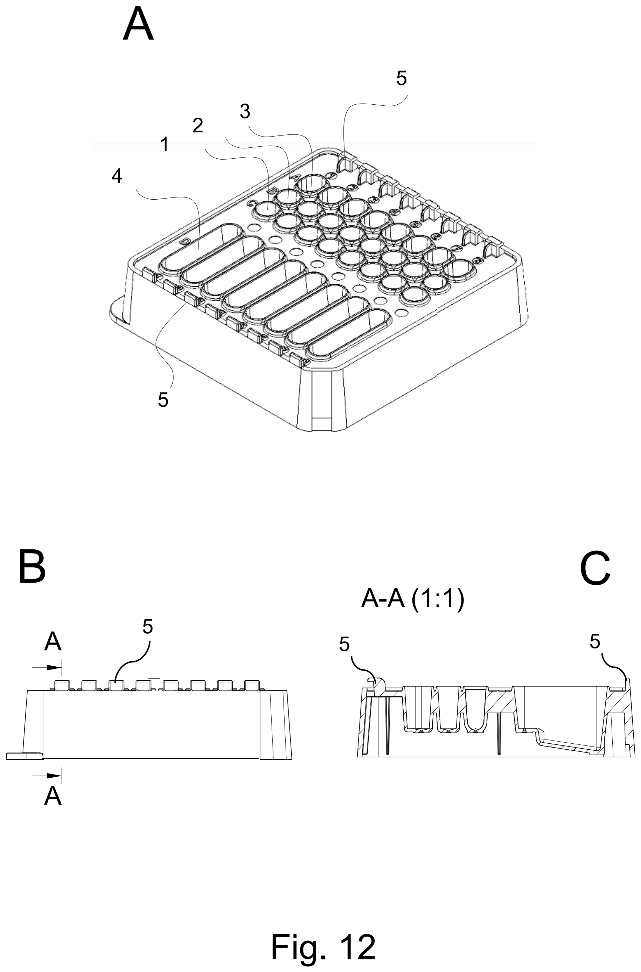

The size of droplets in the emulsion "average cross-sectional dimension" (i.e. average cross-sectional diameter of a perfect sphere having the same volume as the droplet) may be generated in a wide range of dimensions (see definitions), according of their intended form (namely single- or double-emulsion droplets). Single emulsion droplets are typically around 90 µm in diameter; while double-emulsion droplets are about 20 µm in diameter, but larger droplets of about 50 µm in diameter - or even larger - can also be prepared, e.g. by use of a xDrop DE50 cartridge (see example 9 and figure 12) and a Xdrop instrument, (cat. no. IN00100, Samplix ApS, Birkerød, Danmark).

### V. Single-cell resolution of cell killing- and cell secretion-assays

Whereas, very small droplets, of about 25 nm in diameter, are useful for applications directed to selection and/or evolution of chemical molecules from a library of molecules, larger droplets of a diameter of 50 µm or even larger have attracted interest because they allow larger cells to be encapsulated and accordingly opens the avenue to a large number of cellular assays to be performed in particularly analysis of single cells of a population of cells. Figure 12 illustrate a cartridge here referred to as the Xdrop DE50 cartridge. The cartridge is designed to fit into a specially adapted instrument (the Xdrop instrument, cat. no. IN00100, Samplix ApS, Birkerød, Danmark). An embodiment of the instrument is shown in Figure 13. When inserted into the Xdrop instrument and run according to the recommendations of the manufacturers while using the fluorosurfactants of the present invention, the cartridge produces stable double emulsion droplets having a diameter of approximately 50 µm.

In order to perform meaningful cellular assays based on cells encapsulated in the delimited volume of a droplet, it is crucial that the conditions in the droplets remain favorable for cell growth and cell metabolism throughout the incubation period.

Double-emulsion droplets (Water-in-Oil-in-Water) on the other hand may in principle allow various vital components to be exchanged between the aqueous inner milieu of droplets and the outer aquous carrier medium, thereby maintaining homeostasis of the encapsulated cells.

In example 15 we show that double-emulsion droplets formulated with the present fluorosurfactants indeed are permeable to molecules.

We also show that double-emulsion droplets are selective permeable in that the droplet membrane is considerable more permeable to Resorufin than to Fluorescein-Na.

Whereas the MW of Fluorescein-Na (376.27) is comparable to that of Resorufin-Na (235.17), Fluorescein-Na is more hydrophilic than Resorufin. In that respect the artificial fluoride-surfactant stabilised membrane of the present invention fit the so-called Overton rule for biological membranes (Al-Awqati (1999) Nat Cell Biol 8:E201-2) stating that the permeability increases with hydrophobicity. The Overton rule also predicts that dissolved gases such as O₂ and CO₂ will have a high permeability. Anticipating that our artificial membrane comply with the Overton rule and is permeable to the atmosphaere of a CO₂ incubator, seem to offer an explanation to the surprisingly high viability of cells encapsulated in double-emulsion droplets stabilised by the present fluorosurfactants we observe, see e.g. example 14.

As illustrated in example 9, 10, 11, 13, and 14 the tri-block fluorosurfactant stabilise double-emulsion droplets which appears almost non-toxic to encapsulated cells. This opens the avenue for assaying the functionality of one or more cells by encapsulating one or more cells in double-emulsion droplets of a water-in-fluorocarbon oil-in-water emulsion, wherein said fluorocarbon oil emulsion comprises the composition or mixed composition of the invention.

For instance one may assay the cell killing effect of one cell-type (the effector cell) acting upon another type (the target cell) by a method which comprise 1) differential staining of the two cell-types, 2) encapsulate the two cell types in plural double-emulsion droplets forming an double-emulsion, incubate the emulsion of double-emulsion droplets, and 3) perform an analysis of the droplets by flowcytometry.

Example 9 illustrate a method to assay the functionality of one cell-type (the effector cell) acting upon another type (the target cell) comprising the steps:
1) preactivation of effector cells,
2) differential staining of the two cell-types,
3) washing and resuspending cells in a suitable medium,
4) activate effector cells,
5) encapsulate the two cell types in plural double-emulsion droplets forming an double-emulsion, incubate the emulsion of double-emulsion droplets, and
6) perform an analysis of the droplets by flowcytometry.

In example 9 the method is illustrated by interleukin-2 activated natural killer cells ancting upon K562 human erythroleukemia cells.

Since cells survive even prolonged incubation inside the double-emulsion droplets, formulated with the fluorosurfactants of the present invention, living effector cells may be rescued from the droplets that may be expanded. Thus, it is possible by including a suitable stain, staining dead cells (eg. propidium iodide), in the contents of the double-emulsion droplets to sort out droplets with dead target cells, and rescue the effective natural killer cells.

One interesting possibility is to encapsulate CAR-T modified effector-cell together with cancer cells in double emulsion droplets and incubate the emulsion to allow the CAR-T cells to kill the cancer cells. Then may sort-out droplets with dead cancer cells, rescue the desired effective CAR-T cells from droplets, expand the CAR-T cell and use them for immunotherapy.

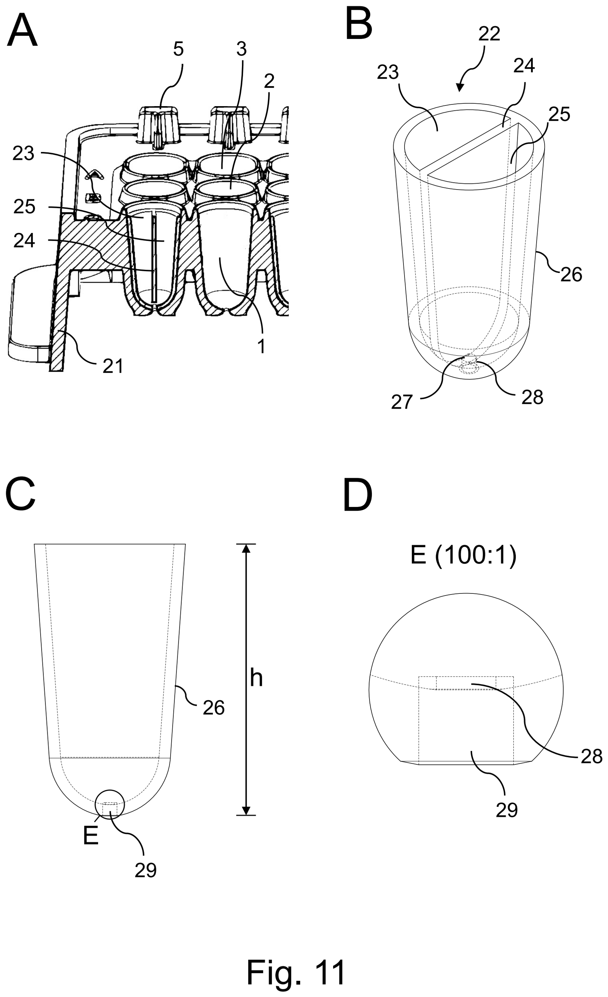

It may be a challenge efficiently to create a large number of droplets which encapsulate just two, in particular two different cells in each droplet. To increase the likelihood of such co-encapsulation the insert described in Figure 11 was applied. The insert, here the split well insert, fits into a sample supply well (1) of a Xdrop^{™} droplet generating cassette (21), e.g. item number CA10100, Samplix ApS, Birkerød, Denmark, or the sample supply well (1) of an a Xdrop DE50 cartrige (Figure 12). When inserted the insert splits the supply well into two chambers (24 and 25) the result being the contents of the chambers are only mixed when they come into contact with each other in the mixing chamber (29), which forms a liquid connection between chamber 1 (23) and chamber 2 (25), and which connects to the primary supply inlet (from the sample supply well) in the droplet generating part of cassette.

It is essential that the two openings (27) and (28) between the two chambers (23) and (25) of the insert have a size and a shape that ensures that the contents (e.g. cell suspensions) of chamber 1 (23) and chamber 2 (25) mixes in a carefully controlled manner and not until the contents of the two chambers are drawn down into the mixing chamber (29) and further into the droplet-producing, microfluidic structure in the droplet-generating cassette.

It has been found that the split well insert greatly increase the formation of droplets with two different cells, one from each of the chambers (23) and (25).

Another interesting application of emulsions stabilised by the fluorosurfactants of the present invention is presented in Example 10.

In this example tri-block fluorosurfactant stabilised droplets are used for analyzing protein-secretion from individual cells.

By encapsulating single cells in double emulsion droplets the cross-talk between cells that otherwise occur in assays with cells in bulk, is avoided.

It is demonstrated that the functionality (ability) at the single-cell-level of a population of cells to excrete a specific substance can be assayed by:
1) suspend the cells with an assay-composition which comprise: a reagent that binds to the surface of the cells and to the specific substance, and an antibody that binds to the same specific substance and which is coupled to a fluorophore.
2) then produce double-emulsion droplets containing cells and assay-components,
3) incubate double-emulsion droplets in a CO₂ incubator,
4) break the droplets to release the cells, and
5) analyse the released cells by flowcytometry.

- and that cellular populations populations are comprised of cells with different potential for stimulation to protein excretion.

In particular the method of Example 10 can be used to evaluate the cellular secretion of two different cytokinins, e.g. INF-y and TNF-α.

The method may also be used to obtain a population of cells excreting a specific substance comprising assaying a population of cells for excreting a specific substance as described while setting the FACS-instrument to collect the population of cells excreting the specific substance, and collect the population of cells excreting the specific substance.

It is noteworthy that although the widespread ICS (Intracellular Cytokine Staining) method (Price, L.S. et al. (2021) Cytometry A.;99:107-116.) is a method acting on cells in bulk, ICS is able to show heterogeneity of cytokine synthesis in a population of cells.

However, as the ICS method imply a cytokine transport inhibitor it can only reveal details of protein ssecertion, not the complete functional action which also include the cross membrane transport. Furthermore, living cells can not be rescued from the ICS assay.

As the cytokine transport inhibitor interferes with normal cell function, it cannot be ruled out that certain cells or secretion of certain cytokines may provide misleading data in the ICS assay. Such data may lead to overinterpretation or underinterpretation of cytokine secretion.

It should be noted that emulsions both of single- or double-emulsion droplets may be formed by use of the fluorosurfactant composition of the invention, employing many of the different droplet formation devises known in the art; for example using the microfluidics cartridge included in the kit described in section VI. Preparation of an emulsion of the invention is exemplified in Example 3 and 5.

### VI. Kit of component parts for forming an emulsion

The invention further provides a kit of component parts suitable for forming an emulsion of single- or double-emulsion droplets. The kit of parts comprises a composition (or mixed composition) comprising a fluorosurfactant according to the invention in a first container; one or more devises for forming an emulsion of single- and/or double-emulsion droplets (also called a microfluidics cartridge), and optionally a fluorocarbon oil in a second container.

The composition in said kit of parts is enriched with either a di-block fluorosurfactant; or a tri-block fluorosurfactant; or the composition is a mixture of compositions enriched respectively with di-block fluorosurfactant; and a tri-block fluorosurfactant, in any desired ratio, for example from 1:1 to 1:50 more preferably 1:1 to 1:20. Preferably the composition or mixed composition is provided as a 5% solution suspended in a hydrofluoroether solvent (fluorinated oil), preferably the HFE-7500/Novec-7500 solvent.

A device (also called a microfluidics cartridge) suitable for forming single- or double-emulsion droplets preferably comprises:
at least one droplet formation unit;
each unit comprising a set of channels that form at least one channel junction,
the set of channels including at least two input channels extending separately from one or more input wells to the channel junction and an output channel extending from the channel junction to an output well, the device being configured to either produce single-emulsion droplets comprising aqueous sample surrounded by an immiscible fluid (e.g. fluorocarbon oil); or to produce double-emulsion droplets comprising an aqueous sample encapsulated within an immiscible fluid (e.g. fluorocarbon oil) and surrounded by a continuous aqueous phase. See figure 8.

### EXAMPLES

### Example 1: Method for synthesizing a diblock fluorosurfactant composition of the invention.

### Reaction Scheme: see figure 1

### Synthesis steps:

### I: Activation of the PFPE chain:

44.0 g of a species of perfluorated PFPE chain having a molecular mass of 7000-7500, and the structure F-[CF(CF₃)CF₂O]_{c}-CF(CF₃)COOH, wherein c is greater than or equal to 30 (also named Krytox 157 FSH, supplied by Chemours, USA), was dissolved in 77.3 g of the solvent Novec HFE-7100 (supplied by 3M, USA).

The carboxy-end group of the perfluorated PFPE-species was activated by addition and reaction with 11.6 g oxalyl chloride ((COCl)₂) in the presence of DMF (N,N-Dimethylformamide) (0.25 ml) at about 26°C for approximately 2½ h, while stirring. The reaction mixture was heated to about 30°C under reduced pressure for approximately 2 h, and the activated perfluorated-species was dissolved in 125 g dried HFE-7100 solvent.

### II. Linking activated PFPE chain to polyetheramine head group

The dissolved activated perfluorated-species was then reacted with a mono-functionalized amine-terminated polyetheramine having a molecular mass of 1000 (also named Jeffamine M1000 supplied by Huntsman Corp., USA). The polyetheramine solution was prepared by dissolving 13.6 g Jeffamine M1000 in an anhydrous mixture of 141 g DCM (dichloromethane, CH₂Cl₂), 2.9 g Et₃N (triethyamine, (C₂H₅)₃N) and 14.3 g 4 Å molecular sieve. The reaction was performed by the dropwise addition of 83 g of a solution of the polyetheramine of under N₂ atmosphere; and the reaction mixture was allowed to proceed at r.t. for approximately 18 h while stirring.

### III: Di-block fluorosurfactant product enrichment

The reaction mixture resulting from step II was passed through a Celite filter, and the filtrate was washed with 1:1 vol HFE-7100. The washed filtrate was concentrated at 50°C under reduced pressure to provide an orange oil. The orange oil was dissolved in a 2:1 mixture of HFE-7500 and MeOH, and the phases were allowed to separate, the lower phase being the product phase. The product phase was further purified by repeating the phase separation step, and the resulting product-phase was passed through a Celite filter, and filtrate was concentrated at 50°C under reduced pressure to provide a composition enriched for the fluorosurfactant.

### IV: Structure of the Di-block fluorosurfactant product

The di-block fluorosurfactant enriched for in the fluorosurfactant composition produced by the above method included species wherein c = 40 - 43; g = 3 and h = 19.

### Example 2: Method for synthesizing a triblock fluorosurfactant composition of the invention.

### Reaction scheme: as show in figure 2

### Synthesis steps:

### I: Activation of the perfluoropolyether (PFPE) chain:

475 g of a species of perfluorated PFPE chain having a molecular mass of 7000-7500, and the structure F-[CF(CF₃)CF₂O]_{c}-CF(CF₃)COOH, wherein c is greater than or equal to 30 (also named Krytox 157 FSH, supplied by Chemours, USA), was dissolved in 830 g of the solvent Novec HFE-7100 (supplied by 3M, USA).

The carboxy- end group of the perfluorated PFPE-species was activated by addition and reaction with 126 g oxalyl chloride ((COCl)₂) in the presence of DMF (N,N-Dimethylformamide) (0.5 ml) at about 22°C for approximately 2½ h, while stirring. The reaction mixture was heated to about 45°C under reduced pressure for approximately 2 h, and the activated perfluorated-species was dissolved in 1350 g dried HFE-7100 solvent.

### II. Linking activated PFPE chain to polyetheramine head group

The dissolved activated perfluorated-species was then reacted with a di-functionalized amine-terminated polyetheramine having a molecular mass of 900 (also named Jeffamine ED-900 supplied by Sigma-Aldrich., USA). The polyetheramine solution was prepared by dissolving 32.7 g Jeffamine ED-900 in an anhydrous mixture of 336 g DCM (dichloromethane, CH₂Cl₂), 47.5 g Et₃N (triethyamine, (C₂H₅)₃N) and 50 g 4 Å molecular sieve. The reaction was performed by the dropwise addition of the polyetheramine solution under N₂ atmosphere; and the reaction mixture was allowed to proceed at about 24°C for approximately 18 h while stirring.

### III: Tri-block fluorosurfactant product enrichment

The reaction mixture resulting from step II was passed through a glass fiber filter, and the filtrate was washed with 1:1 vol HFE-7100. The washed filtrate was concentrated at 50°C under reduced pressure to yield an orange oil. The orange oil was dissolved and shaken in a 2:1 mixture of HFE-7500 and MeOH, and the phases were allowed to separate, the lower phase being the product phase. The product phase was further purified by repeating the phase separation step, and the resulting product-phase was passed through a glass fiber filter, and filtrate was concentrated at 50°C under reduced pressure to provide a composition enriched for the fluorosurfactant.

### IV: Structure of the Tri-block fluorosurfactant product

The tri-block fluorosurfactant enriched for in the fluorosurfactant composition produced by the above method included species wherein c = 40 - 43; d + f = 6 and e = 12.5

### Example 3: Method for producing an emulsion of double-emulsion droplets

Double emulsion droplets (Water-in-Oil-in-Water) were created using Xdrop dPCR proprietary droplet production technology as described by Madsen et al., 2020 (Human mutation doi: 10.1002/humu.24063).

The dPCR droplets were produced on an Xdrop instrument (cat. no. IN00100, Samplix ApS, Herlev, Denmark) using the double-emulsion generating cartridge (Samplix cat. no. CA10100) and standard run parameters (production time of 40 minutes).

Briefly, an aqueous 1xdPCR buffer and 1xPCR mix (from Samplix dPCR-kit, cat. no. RE 10100, and diluted 1X according to instructions), a fluorocarbon oil (Novec 7500, 3M Co., Maplewood, MN, USA), and a fluorosurfactant (comprising of one or more compositions produced by the method of the invention) were used, as detailed below. The double-emulsion cartridge was prepared by loading:
1. 300 µl 1x dPCR buffer in the first well (#A),
2. 40 µl 1x dPCR buffer to the shelf of the outlet well #D,
3. 40 µl 1x dPCR-mix into well #C,
4. 100 µl fluorosurfactant mixture into well #B;
   and then
5. Placing a gasket to the top of the cartridge; and
6. Inserting the cartridge into the Xdrop instrument, and running the "dPCR" program installed on the instrument.
7. When finished, the double emulsion droplets were collected from the outlet well (#D).

The fluorosurfactant mixture was either a composition comprising the di-block fluorosurfactant (A-X-B) or a composition comprising the tri-block fluorosurfactant (AX-B-X-A), each produced by the method of the invention; or a combination of the two compositions diluted in Novec 7500. 12 different mixtures of fluorocarbon oil with fluorosurfactants were made and tested, see table 1:

| Table1. Fluorosurfactant mixures | | | | | |
|---|---|---|---|---|---|
| Experim ent | Fluorosurfactant (FS) | % w/v FS, final | Triblock, ul | Diblock, ul | Novec 7500, ul |
| 1 | Triblock* | 5 | 400 | - | 0 |
| 2 | Triblock* | 3 | 240 | - | 160 |
| 3 | Triblock* | 1 | 80 | - | 320 |
| 4 | Diblock** | 5 | - | 400 | 0 |
| 5 | Diblock** | 3 | - | 240 | 160 |
| 6 | Diblock** | 1 | - | 80 | 320 |
| 7 | Tri/di 9:1 | 5 | 360 | 40 | 0 |
| 8 | Tri/di 9:1 | 3 | 216 | 24 | 160 |
| 9 | Tri/di 9:1 | 1 | 72 | 8 | 320 |
| 10 | Tri/di 8:2 | 5 | 320 | 80 | 0 |
| 11 | Tri/di 8:2 | 3 | 192 | 48 | 160 |
| 12 | Tri/di 8:2 | 1 | 64 | 16 | 320 |
| 13 | No surfactant | 0 | 0 | 0 | 400 |

| | | | | | |
|---|---|---|---|---|---|
| *Tri-block structure: A-X-B-X-A is F-[CF(CF₃)CF₂O]_{c}-CF(CF₃)CONH-[CH(CH₃)CH₂O]_{d}-[CH₂CH₂O]ₑ-[CH(CH₃)CH₂O]_{f}-CH₂CH(CH₃)NHCOCF(CF₃)-[OCF₂CF(CF₃)]_{c}-F, wherein c≈41; d≈3, e≈12,5 and f≈3 **Di-block structure: (A-X-B) is F-[CF(CF₃)CF₂O]_{c}-CF(CF₃)CONH-[CH(CH₃)CH₂O]_{g}-[CH₂CH₂O]ₕ-CH₃, wherein c≈41; g≈3 and h≈19. | | | | | |

The PCR-mix composition see table 2

| Table 2. Composition of PCR-mix | |
|---|---|
| Reagents | 1 reaction [µl] |
| dPCR mix (2x) | 20 |
| Forward primer (10µM) | 0,8 |
| Reverse primer (10µM) | 0,8 |
| H2O | 16,4 |
| Total mix | 38 |
| Template SD1111 | 2 |
| Total volume | 40 |

Template was Jurkat genomic DNA, cat # SD1111 (Thermo-Fisher, Waltham, MA, USA). Primers were 20-nucleotide standard primers directed against TP53 (Eurofins, Glostrup, Denmark).

### RESULT

Droplet-formation, see table 3.

| Table 3. Result droplet formation | | | | |
|---|---|---|---|---|
| | Fluorosurfactant (FS) | % FS total | Droplets formed | Comments |
| 1 | Triblock | 5 | Yes | |
| 2 | Triblock | 3 | Yes | |
| 3 | Triblock | 1 | Yes | Some bigger structures w multiple cores |
| 4 | Diblock | 5 | Yes | Some bigger structures w multiple cores, particles in shell |
| 5 | Diblock | 3 | Yes | |
| 6 | Diblock | 1 | Yes | Thick shelled |
| 7 | Tri/di 9:1 | 5 | Yes | |
| 8 | Tri/di 9:1 | 3 | Yes | |
| 9 | Tri/di 9:1 | 1 | Yes | But few |
| 10 | Tri/di 8:2 | 5 | Yes | |
| 11 | Tri/di 8:2 | 3 | Yes | |
| 12 | Tri/di 8:2 | 1 | Yes | Some bigger structures w multiple cores |
| 13 | Novec* | 0 | No | Failed, only foam and sticky-sticky |

| | | | | |
|---|---|---|---|---|
| *Control where no fluorosurfactant was added to the fluorinated Novec 7500 oil. | | | | |

The formation of double-emulsion droplets using each of the fluorosurfactant composition/mixed compositions of Table 1 are shown in Table 3, and figure 9. Note a number of lipid-drops are also seen in particular in the preparations comprising only 1 % fluorosurfactant in the fluorosurfactant mixure.

### CONCLUSION

This data demonstrates that use of a 1 - 5% fluorosurfactant composition of the triblock fluorosurfactant (A-X-B-X-A) alone, the di-block fluorosurfactant (A-X-B) alone, as well as combinations of the di-block and tri-block fluorosurfactant compositions facilitated production of double-emulsion droplets. This shows that a fluorosurfactant is an absolute requirement for production of stable emulsions.

### Example 4: Stability and "bio"-compatibility of double-emulsion droplets during PCR conditions and FACS.

The stability of the double-emulsion droplets made in example 3 were tested by subjecting the droplets comprising PCR reagents to thermocycling, using the two programs in Table 4 with either 40 or 60 repetitions, as set out below:

| Table 4. PCR on droplets | | | |
|---|---|---|---|
| | Temperature | Duration | Repetitions |
| 1 | 30°C | 5 sec | 1x |
| 2 | 94°C | 2 min (slow ramp 1.5 C/sec) | 1x |
| 3 | 94°C | 3 sec (slow ramp 1.5 C/sec) | 40x or 60x |
| 4 | 60°C | 30 sec (slow ramp 1.5 C/sec) | |
| 5 | 24°C | 30 sec | 1x |

Stability of the emulsions was evaluated by visual inspection in a microscope.

The result is reported in table 5.

| Table 5. Droplet stability after thermocycling (60 repetitions) | | | | |
|---|---|---|---|---|
| | Fluorosurfactant (FS) | % FS total | Stable droplets | Comments |
| 1 | Triblock | 5 | Yes | |
| 2 | Triblock | 3 | Yes | |
| 3 | Triblock | 1 | Yes | Some larger |
| 4 | Diblock | 5 | Yes | |
| 5 | Diblock | 3 | Yes | |
| 6 | Diblock | 1 | Yes | Some larger structures, DE w thick shells |
| 7 | Tri/di 9:1 | 5 | Yes | Some larger |
| 8 | Tri/di 9:1 | 3 | Yes | |
| 9 | Tri/di 9:1 | 1 | Yes | |
| 10 | Tri/di 8:2 | 5 | Yes | |
| 11 | Tri/di 8:2 | 3 | Yes | Fewer DE |
| 12 | Tri/di 8:2 | 1 | Yes | Thick shells |
| 13 | Novec | 0 | No | No droplets were formed |

| | | | | |
|---|---|---|---|---|
| Novec* is HFT 7500 without fluorosurfactant. | | | | |

The droplets formed by 1, 3 and 5% w/v tri-block fluorosurfactant in fluorocarbon oil and amplified by 40 cycles of PCR were further analysed by FACS.

*Stable droplet formation is defined as formation of double-emulsion (DE) droplets, wherein the %'s of DE-droplets with a diameter 2 times larger than the average diameter of the DE-droplets (coalescent droplets) is less than 2%.

The positive identification of droplets in the FACS procedure is based on the detection of PCR-amplified DNA by staining the DNA with DNA-specific fluorescent stains before the onset of the FACS procedure.

A Sony Cell Sorter SH800S instrument equipped with a 100 µm nozzle was used for the sorting. Approximately 4 mill. events were recorded, positive droplets were sorted in emulsions formed with both 1, 3 and 5% w/v tri-block fluorosurfactant in fluorocarbon oil, indicating that the droplets were sufficient stable to withstand the conditions during the FACS analysis, and indicate that the tri-block fluorosurfactant did not inhibited the PCR-enzymatic reaction to any significant degree. The result is summarised in Table 6.

| Table 6. FACS analysis | | | |
|---|---|---|---|
| | FACS ex. 1 | FACS ex. 2 | FACS ex. 3 |
| Fluorosurfactant (FS) | 5% triblock | 3% triblock | 1% triblock |
| PCR type | std PCR 40 cycles | std PCR 40 cycles | std PCR 40 cycles |
| Nozzle | 100 µm | 100 µm | 100 µm |
| Positive droplets sorted | 135 | 277 | 326 |
| Total number of events | 4.253.571 | 4.242.406 | 6.474.713 |

### CONCLUSION

Both the tri-block fluorosurfactant (A-X-B-X-A) alone, di-block fluorosurfactant (A-X-B) alone, as well as combinations of the di-block and tri-block fluorosurfactant facilitated production of double-emulsion droplets which were sufficiently stable to perform PCR thermocycling with at least 60 repetitions .

A fluorosurfactant content of between 1 and 5% w/v in the Novec 7500 fluorocarbon oil produced stable water-oil-water emulsions.

From the visual inspection it appeared that a 3% w/v was superior to a 1% w/v composition of fluorosurfactant in fluorocarbon oil.

Both 1, 3 and 5% w/v tri-block fluorosurfactant compositions produce droplets that are sufficient stable to perform a FACS analysis.

The tri-block fluorosurfactant did not inhibited the PCR-enzymatic reaction to any significant degree.

### Example 5: Method for producing an emulsion of single-emulsion droplets

Single emulsion dMDA droplets (Water-in-Oil) were created using Xdrop dMDA proprietary droplet production technology as described by Madsen et *al.,* 2020 (Human mutation doi: 10.1002/humu.24063).

The dMDA droplets were produced on an Xdrop instrument (cat. no. IN00100, Samplix ApS, Herlev, Denmark) using the single-emulsion generating cartridge (Samplix cat. no. CA20100) and standard run parameters (production time of 1 minute).

Briefly, dMDA mix (from Samplix dMDA-kit, cat. no. RE20300) containing hexamer primers and dNTPs, a phi29 enzyme (from Samplix dMDA-kit, cat. no. RE20300), a fluorocarbon oil component (Novec 7500, 3M Co., Maplewood, MN, USA), and a fluorosurfactant component (comprising of one or more compositions produced by the method of the invention) were used.

The single-emulsion cartridge was prepared by loading:
1. 20 µl dMDA mastermix with template in the inlet well with bore tips. The mixture moved directly into the cartridge lane and did not stay in the inlet well;
2. 75 µl fluorosurfactant mixture in the inlet well with a regular tip. The fluorosurfactant mixture stayed in the inlet well until droplet formation was initialized in the Xdrop instrument;
   and then
5. Placing a gasket to the top of the cartridge.
6. Inserting the cartridge into the Xdrop instrument, and running the "dMDA" program installed on the instrument.
7. When the program was completed the single emulsion droplets were collected from the outlet well.

The fluorosurfactant mixture was either a composition comprising the di-block fluorosurfactant (A-X-B) or a composition comprising the tri-block fluorosurfactant (AX-B-X-A), each produced by the method of the invention; or a combination of the two compositions. 6 different mixtures of fluorocarbon oil with fluorosurfactants was made and tested, see table 7

| Table 7. Fluorosurfactant mixtures. All mixtures had a final FS concentration of 5 % w/v. | | | | | |
|---|---|---|---|---|---|
| Experi ment | Triblock*, ul | Triblock* % | Diblock**, ul | Diblock** % | Novec 7500, ul |
| 1 | 400 | 5.0 | 0 | 0.0 | 7600 |
| 2 | 360 | 4.5 | 40 | 0.5 | 7600 |
| 3 | 320 | 4.0 | 80 | 1.0 | 7600 |
| 4 | 280 | 3.5 | 120 | 1.5 | 7600 |
| 5 | 240 | 3.0 | 160 | 2.0 | 7600 |
| 6 | 200 | 2.5 | 200 | 2.5 | 7600 |

| | | | | | |
|---|---|---|---|---|---|
| *Tri-block structure: A-X-B-X-A is F-[CF(CF₃)CF₂O]_{c}-CF(CF₃)CONH-[CH(CH₃)CH₂O]_{d}-[CH₂CH₂O]ₑ-[CH(CH₃)CH₂O]_{f}-CH₂CH(CH₃)NHCOCF(CF₃)-[OCF₂CF(CF₃)]_{c}-F, wherein c≈41; d≈3, e≈12,5 and f≈3 **Di-block structure: (A-X-B) is F-[CF(CF₃)CF₂O]_{c}-CF(CF₃)CONH-[CH(CH₃)CH₂O]_{g}-[CH₂CH₂O]ₕ-CH₃, wherein c≈41; g≈3 and h≈19. | | | | | |

The dMDA mastermix composition including template see table 8.

| Table 8. Composition of dMDA mastermix including template | |
|---|---|
| Reagents | 1 reaction [µl] |
| dMDA mix | 4 |
| H2O | 5 |
| dMDA enzyme (phi29) | 1 |
| Total mix | 10 |
| Template | 10 |
| Total volume | 20 |

The DNA template was Jurkat genomic DNA, cat # SD1111 (Thermo-Fisher, Waltham, MA, USA).

### RESULT

Droplet-formation, see table 9.

| Table 9. Result droplet formation | | | |
|---|---|---|---|
| | % Triblock | % Diblock | Droplet formation |
| 1 | 5.0 | 0.0 | Yes |
| 2 | 4.5 | 0.5 | Yes |
| 3 | 4.0 | 1.0 | Yes |
| 4 | 3.5 | 1.5 | Yes |
| 5 | 3.0 | 2.0 | Yes |
| 6 | 2.5 | 2.5 | Yes |

### CONCLUSION

The results show that both a composition of the tri-block fluorosurfactant (A-X-B-X-A) alone, and various combinations of the tri-block andh di-block fluorosurfactant (A-X-B) compositions facilitated production of single-emulsion droplets.

### Example 6: Stability and "bio"-compatibility of single-emulsion droplets during dMDA.

The stability of the single-emulsion droplets made in Example 5 was tested by subjecting the droplets comprising dMDA reagents to 16 hours of incubation at 30 °C followed by 10 minutes of incubation at 65 °C. "Break solution" (from Samplix dMDA kit, cat. no. RE20300) and "Break colour" (from Samplix dMDA kit, cat. no. RE20300) were mixed with the dMDA products, in order to break the single-emulsion droplets and release the DNA into solution.

Subsequently, all break solution was removed from the DNA solution; and Quantus dye (from cat. no. E4871, Promega, Madison, WI, USA) and TE buffer were mixed with 1 µl sample of the DNA solution, and then incubated at room temperature in darkness for 5 minutes, whereafter the concentration of DNA in the 1 µl sample was quantified on a Quantus instrument (cat. No. E6150, Promega, Madison, WI, USA).

### RESULTS

The DNA concentration following dMDA amplification performed on droplets prepared in Example 5 is shown in Table 10.

| Table 10. Resulting DNA concentrations from dMDA reactions | | | |
|---|---|---|---|
| Experiment | Template | Volume after break (µl) | DNA concentration (ng/µl) |
| 1 | 1 pg Jurkat DNA | >10 | 114 |
| 2 | 1 pg Jurkat DNA | >10 | 105 |
| 3 | 1 pg Jurkat DNA | >10 | 100 |
| 4 | 1 pg Jurkat DNA | >10 | 106 |
| 5 | 1 pg Jurkat DNA | >10 | 113 |
| 6 | 1 pg Jurkat DNA | >10 | 114 |

### CONCLUSION

The NCK fluorosurfactant stabilized the dMDA droplets and the droplets remained intact after 16 hours at 30 °C followed by 10 min at 65 °C.

All the DNA measurements were high (>100 ng/µl) which indicates that there was little or no inhibition of the dMDA reaction by the fluorosurfactant.

No effect was observed of changing the ratio of di- and tri-block fluorosurfactants.

### Example 7: Assay for stability properties of fluorosurfactant

The stability properties of fluorosurfactant compositions was assayed by determining single-emulsion monodispersity (lack of coalescence) after droplet formation (t=0) and after incubation for 16 h at 30°C followed by 10 minutes of incubation at 65°C.

The single-emulsion droplets were prepared as described in example 5.

The dMDA droplet monodispersity after dMDA droplet formation (t=0) and after dMDA incubation was evaluated by:
1. Resuspending the emulsion by gently flicking the PCR-tube with dMDA droplets.
2. Pipetting gently 5 µl of the emulsion into a counting chamber (Bürker-Türk, Counting Chambers, depth: 0.100 mm) underneath the cover glass.
3. Adding 5 µl dMDA oil under the cover glass
4. Using a 4x microscope objective to find an area with droplets lying in a single layer and where the droplets cover the visible area.
5. Taking a bright-field image of the droplets
6. Measuring the diameter of 5 droplets that are in the size range of the majority of droplets, and determinig the average size of the 5 droplets.
7. Counting the number of droplets with a diameter 2x or more than the average diameter of droplets measured.
8. Estimating the number of droplets in the image by multiplying the number of droplets on the y-axis with the number on the x-axis. Typically it this will be around 900 dMDA droplets.

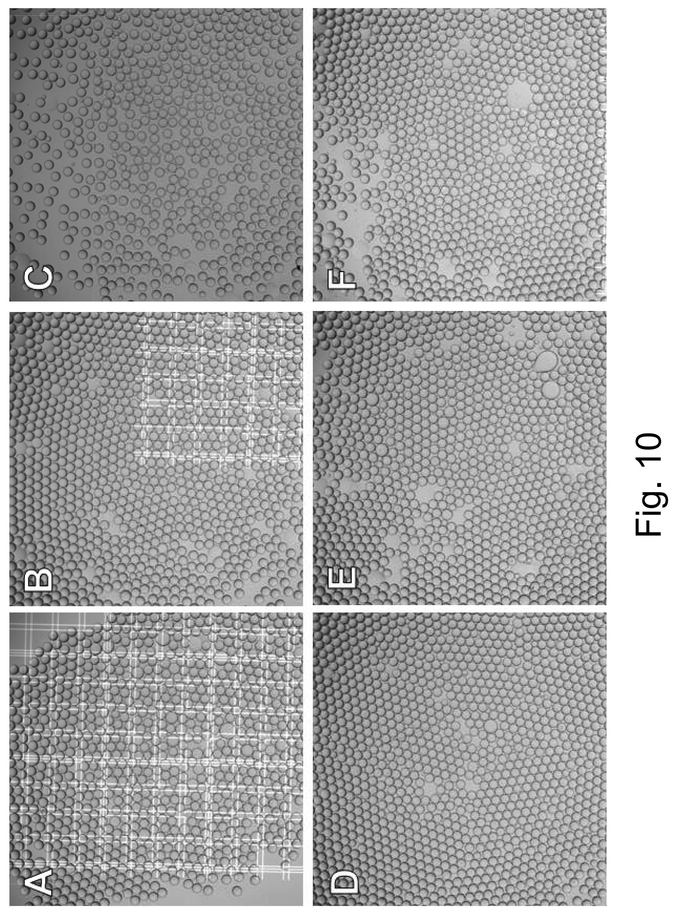

Figure 10 is a typical result of the stability testing assay. It shows the single-emulsion droplets formed in example 5 as they appear in a bright-field image after incubation for 16h at 30°C followed by 10 minutes of incubation at 65°C.

All emulsions prepared in Example 5 and after the dMDA incubation step had less than 2% droplets with a diameter 2x larger than the average diameter of the droplets.

### Example 8: Assay for stability properties of fluorosurfactant during thermocycling

The stability properties of fluorosurfactant compositions was also assayed by determining single-emulsion monodispersity (lack of droplet coalescence) after droplet formation and incubation under PCR-conditions, see table 14.

| Table 14. PCR | | | |
|---|---|---|---|
| | Temperature | Duration | Repetitions |
| 1 | 30°C | 5 sec | 1x |
| 2 | 94°C | 2 min (slow ramp 1.5 C/sec) | 1x |
| 3 | 94°C | 3 sec (slow ramp 1.5 C/sec) | 40x or 60x |
| 4 | 60°C | 30 sec (slow ramp 1.5 C/sec) | |
| 5 | 24°C | 30 sec | 1x |

The single emulsion droplets were prepared by the method described in example 5 except that a PCR-mix (see table 15) was used instead of a dMDA mastermix.

| Table 15. dPCR mastermix preperation | | | | | |
|---|---|---|---|---|---|
| exp | Reagents* | 1 reaction [µl] | **Final** concentration [µM] | Total reactions [µl] | 1 reaction [µl] |
| 1 | dPCR mix (2x) | 10 | 1x | 85 | 10 |
| 2 | Forward primer (10µM) | 0.4 | 0.2 | 3.4 | 0.4 |
| 3 | Reverse primer (10µM) | 0.4 | 0.2 | 3.4 | 0.4 |
| 4 | EvaGreen (20x) | 1 | | 8.5 | 1 |
| 5 | H2O | 7.2 | | 61.2 | 7.2 |
| 6 | Total mix | 19 | | 161.5 | 19 |
| 7 | Template (Jurkat 10ng/ul) | 1 | | 8.5 | 1 |
| 8 | Total volumen | 20 | | 170 | 20 |

| | | | | | |
|---|---|---|---|---|---|
| *Reagents see Example 3. | | | | | |

The fluorosurfactant mixure was either a composition comprising the di-block fluorosurfactant (A-X-B) or a composition comprising the tri-block fluorosurfactant (AX-B-X-A), each produced by the method of the invention; or a combination of the two compositions. 8 different mixtures of fluorocarbon oil with fluorosurfactants was made and tested, see table 16.

| Table 16. Fluorosurfactant mixing | | | | | |
|---|---|---|---|---|---|
| exp | Fluorosurfactant (FS) | % FS | Triblock, µl | Diblock, µl | Novec 7500, µl |
| 1 | Triblock | 5 | 400 | - | 0 |
| 2 | Triblock | 3 | 240 | - | 160 |
| 3 | Diblock | 5 | - | 400 | 0 |
| 4 | Diblock | 3 | - | 240 | 160 |
| 5 | Tri/di 9:1 | 5 | 360 | 40 | 0 |
| 6 | Tri/di 9:1 | 3 | 216 | 24 | 160 |
| 7 | Tri/di 8:2 | 5 | 320 | 80 | 0 |
| 8 | Tri/di 8:2 | 3 | 192 | 48 | 160 |

Eight single emulsion droplet-preparations were prepared as described (example 5), and the droplets were characterised.

Figure 10 is a typical result of the stability testing assay. It shows the single-emulsion droplets formed in example 5 as they appear in a bright-field picture after incubation for 16h at 30°C followed by 10 minutes of incubation at 65°C.

All single-emulsions (prepared using techniques of Example 5) and after the PCR thermocycling step had less than 2% droplets with a diameter 2x larger than the average diameter of the droplets.

### Example 9: Tri-block fluorosurfactant droplets can be used for analyzing cell-cell killing activity of mammalian cells

In this example, it is demonstrated that the tri-block fluorosurfactant can be used for stabilizing double emulsion droplets while still allowing cell viability. A system using the surfactant is used to demonstrate cell-cell killing activity.

Workflow, se figure 14 for an overview:
1) Mix natural killer (NK) cells with a green fluorescent dye and propidium iodide which stain dead cells only.
2) Mix target cells with dark red fluorescent dye and propidium iodide.
3) Produce double-emulsion droplets containing both killer cells and target cells.
4) Incubate double-emulsion droplets in a CO₂ incubator.
5) Analyze droplets by flow-cytometry.

Three preparations of:
1) 1×10⁶ NK-92 (Cat. no. ACC 488, DSMZ-German Collection of Microorganisms and Cell Cultures GmbH, Braunschweig, Germany), cultured natural killer cells were preactivated by incubation for 24 hours with 100ng/ml IL-2 (Cat. no. 130-097-742, Miltenyi Biotec, Teterow, Germany for 24 hours prior),
2) 1×10⁶ NK-92 cells (maintained in a non-activated stage in medium with 5ng/ml IL-2), and
3) 6×10⁶ K562 ((Cat. no. ACC 10, DSMZ)
   - were collected in 3 separate tubes, pelleted and resuspended in Dulbecco's Phosphate Buffered Saline (DPBS).

Staining of cells: CellTrace^{™} CFSE dye (Cat. no. C34554, Invitrogen, MA, USA) dye was added to the both NK-92 cell suspensions, and eBioscience^{™} Cell Proliferation Dye eFluor^{™} 670 (Cat. no. 65-0840-85, Invitrogen, MA, USA) was added to the K562 cells. The cells were stained for 20 min at 37°C. After incubation, the cells were washed and resuspended in α-MEM media (Cat. no. 12571063, Gibco, MA, USA) with 12.5% FBS Fetal bovine serum (Cat. no. F9665, Sigma-Aldrich, MI, USA) and 12.5 horse serum (Cat. no. H1138, Sigma-Aldrich, MI, USA), 1% Penicilin-Streptomycin (P/S) (Cat. no. 15140122, Gibco, MA, USA), 1µg/ml Propidium Iodide (PI) (cat. no. BMS500PI, ThermoFisher Scientific, MA, USA) and 10% optiprep^{™} (Cat. no. 07820, Stemcell Technologies, Vancouver, Canada). 100ng/ml IL-2 was added to the preactivated NK-92 cells.

Droplet production: NK-92 cells were loaded into one side of a split sample well insert (Danish utility model BA2021-00110, and figure 11) inserted into an Xdrop DE50 dropforming cartridge (see Figure 12) to give an average concentration of 1 cells/droplet. Then K562 cells were loaded into the other side of the split sample well insert to give a final concentration of 2 cells/droplet. The tri-block fluorosurfactant surfactant was mixed 5% w/w into NOVEC7500 (3M^{™}), and the oil containing surfactant was loaded into the oil inlet well of the cartridge. Cell culture medium (a-MEM media with 12.5% FBS and 12.5 horse serum, 1% P/S and 10% optiprep^{™}) mixed with stabilizing solution for cells 3X (Samplix, Birkerød, Denmark, cat # REDIVSTABSOL1500) was loaded into the outer medium inlet of the cartridge and double emulsion droplets containing both types of cells were produced by inserting the cartridge into an Xdrop instrument (cat. no. IN00100, Samplix ApS, Birkerød, Denmark, fig. 13) and starting droplet production closely following the recommandations of the manufacturer.

Two droplet-productions were made:
I) Tubes 1) and 3) mixed 1:1 by use of the split well insert in one droplet-production, may be referred to as the "Activated assay".
II) Tubes 2) and 3) are mixed 1:1 by use of the split well insert in a second droplet-production, referred to as the "Non-activated assay".

Incubation: The 2 productions (I and II) were divided into 4 portions each, one for each time point (1, 2, 4 and 24 hours) and left in the incubator (Forma Steri-Cycle i250, ThermoFisher Scientific, MA, USA) at 37°C with , 5% CO₂) until flow-cytometric analysis.

Flow-cytometry: was performed using a BD Accuri flow cytometer (Beckton Dickinson, San Diego, California, cat # 660517) using the blue and red lasers (488 nm and 640nm, respectively) and 533/30, 670LP, 675/25 filter. The analysis was done using FCS Express software (De Novo Software, Pasadena, California, version 7.12.0007).

### RESULT

Flow-cytometric analysis of the double emulsion droplets showed six distinct populations corresponding to droplets with 1) no cells, 2) one K562 cell, 3) two K562 cells, 4) one NK-92 cell, 5) one NK-92 cell and one K562 cell, and 6) one NK-92 cell and two K562 cells. When gating around the different populations and analyzing for propidium iodide stain (staining dead cells), it was found that viability in droplets with only K562 cells or NK-92 cells was high, whereas viability was significantly lower in droplets with both NK-92 and K562 cells. The assay successfully showed killing of target cells by individual killer cells (Figure 15).

### CONCLUSION

The data presented in figure 15 show that the tri-block fluorosurfactant can be used for stabilizing fluorocarbon oil double-emulsion droplets. Viability of control cells (K562 cells or NK cells in droplets with no other cells) was close to 90% after 24 hours of incubation showing that the surfactant is very compatible with cell viability. Droplets with both a target K562 cell and an effector NK cell showed much lower viability because of the killing of target cells. This finding was confirmed by fluorescence microscopy. These data show that the NK cells are not only viable but also able to kill target cells when they are inside the droplets produced using the tri-block fluorosurfactant.

### Example 10: Tri-block fluorosurfactant stabilised droplets used for analyzing protein-secretion from individual cells

In this example, it is demonstrated that a system using tri-block fluorosurfactant stabilised droplets can be used to analyze the protein secretion from individual mammalian cells.

### Workflow:

1) Mix cells with an assay containing
   a. A reagent that binds to the surface of the cells and to an interleukin
   b. An antibody that binds to the same interleukin and which is coupled to a fluorophore.
2) Produce double-emulsion droplets containing cells and assay-components
3) Incubate double-emulsion droplets in a CO₂ incubator
4) Break the droplets to release the cells, and
5) Analyze cells on a flow cytometer. Only cells secreting the specific interleukin will bind the fluorescently labelled antibody on the surface of the cells and can be identified as such on a flow cytometer.

Workflow is illustrated in figure 17.

Assay components: NK-92 cells or Human Peripheral Blood Mononuclear Cells (PBMCs) were collected, washed (spun down 300g for 5min, cell pellet resuspended in DPBS and spun down again and resuspended in media), and labelled with either INF-y or TNF-α binding antibodies (cat. no. 130-090-433 and 130-091-268, Miltenyi Biotec, Teterow, Germany) for 20 min at 4°C. Excess reagents was removed by washing as before and the cells were resuspended in media (NK-92: α-MEM media with 12.5% FBS and 12.5 horse serum and 1% P/S, PBMC: RPMI-1640 (cat. no. 52400041, Gibco, MA, USA) with 10% FBS and 1% P/S. 10% Optiprep^{™} and the secondary fluorescent INF-y and TNF-α antibodies (cat. no. 130-090-433 and 130-091-268, Miltenyi Biotec, Teterow, Germany) were added just prior to droplet production.

Activation: The NK-92 cells were activated by adding 100ng/ml IL-2 (Cat. no. 130-097-742, Miltenyi Biotec, Teterow, Germany) prior to encapsulation, and activation of the PBMC was done by adding eBioscience^{™} Cell Stimulation Cocktail (500X) (cat no. 00-4970-93, Thermo Fisher Scientific, MA, USA) just prior to encapsulation.

Droplet production: In case of both the IFNg and the TNFa assay NK-92 cells or PBMCs with was loaded into the sample well of a cartridge that produces stable double emulsion droplets having a diameter of approximately 50 µm, the DE50 cartridge (see figure 12) to give an average concentration of 0.2 cells/droplet. The tri-block 00fluorosurfactant was mixed to form a 5% w/w Novec-7500 solution. Then the surfactant solution was loaded into the oil inlet well of the DE50 cartridge. Cell culture medium (NK-92: α-MEM media with 12.5% FBS and 12.5 horse serum, 1% P/S and 10% optiprep^{™}, PBMC: RPMI-1640 with 10% FBS, 1% P/S and 10% Optiprep^{™}) and stabilizing solution for cells ((Samplix, Birkerød, Denmark, cat # REDIVSTABSOL1500) were loaded into the outer medium inlet of the cartridge and double emulsion droplets containing both types of cells were produced by inserting the cartridge into a Xdrop instrument (figure 13, cat. no. IN00100, Samplix ApS, Birkerød, Danmark) and droplets were formed following the recommendations of the manufacturers.

The droplet-productions were then transferred to an CO₂-incubator (Forma Steri-Cycle i250, ThermoFisher Scientific, MA, USA) and incubated for 3 hours (NK-cells) and 4 hours (PBMC) at 37°C, 5% CO2.

Droplet breaking and recovery of cells: The outer media in the droplets were first exchanged with DPBS (Dulbecco's Phosphate Buffered Saline) supplemented with 1% bovine serum albumin (BSA) and 1% EDTA and then broken down by adding break solution (from Samplix, Birkerød, Denmark dMDA kit, cat. no. RE20300). Cells are recovered by mixing, and giving mix a shot spin which result in that the mixture divides into 2 phases, break solution and oil is in the lower and the cells stay in the DPBS top phase. The cells are recovered from the DPBS top phase.

The free cells in the DPBS were transferred to clean tubes, washed (2X spun down, spun down 300g for 5min, and resuspended in medium) and poststained for 20 min at 4°C with LIVE/DEAD^{™} Fixable Green Dead Cell Stain Kit (cat. no. L23101, Invitrogen, MA, USA) and the PBMCs was stained for CD3 (cat. no. 345766, BD, NJ, USA), a marker for T-cells.

Flow cytometry was performed using a BD Accuri flow cytometer (Beckton Dickinson, San Diego, California, cat # 660517) using the blue and red lasers (488nm and 640nm, respectively) and the 533/30, 585/40, 670LP, 675/25 filters and analysis was done using FCS Express software (De Novo Software, Pasadena, California, version 7.12.0007).

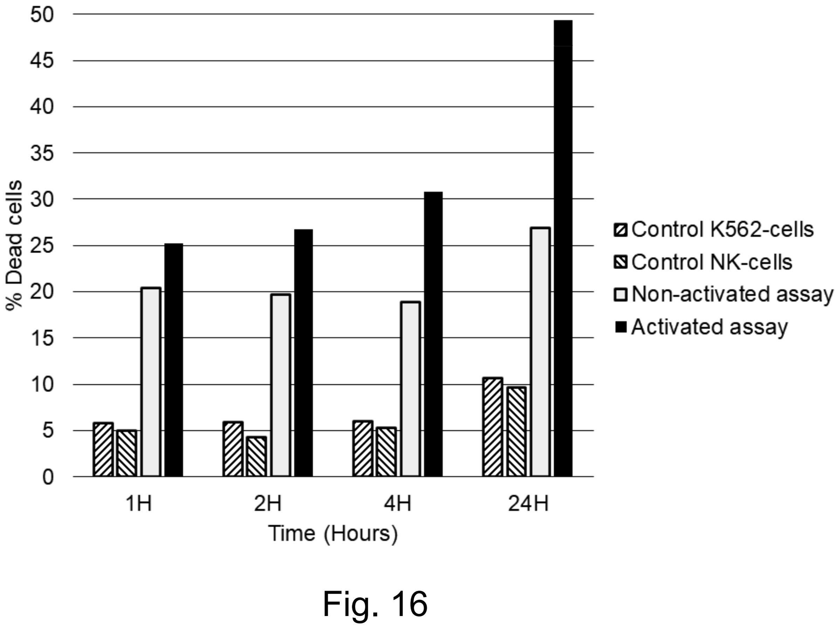

Flow cytometry analysis of the activated cells recovered from double-emulsion droplets showed that the cells were composed of populations with very high cytokine secretion activity as well as populations with very low activity, see figure 15. The similar assay performed in bulk (figure 16) showed a distribution of cells that appear as only one major population. The droplets allow each of the cells to be assayed individually and to reveal that in fact the the cells forms a mixture of different cell populations some of which forms a population of highly potent cells. This population of highly potent cells is not obvious in cells activated in bulk.

### CONCLUSON

The data presented in figure 16 show that the tri-block fluorosurfactant can be used for stabilizing fluorocarbon oil double-emulsion droplets. By encapsulating single cells in double emulsion droplets the cross-talk between cells that otherwise is unavoidable in assays with cells in bulk is avoided. The single-cell approach of the present method allow one to realise that populations both of NK-92 cells and of Human Peripheral Blood Mononuclear Cells (PBMCs) are comprised of cells with different potential for stimulation of in casu for INF-y and for TNF-α secretion.

**Example 11:** The tri-block fluorosurfactant provides high stability to double emulsion droplets.

Double emulsion droplets (Water-in-Oil-in-Water) were made essentially as described in example 3.

Specifically, Cell culture medium (in case of NK-92 cells: α-MEM media with 12.5% FBS and 12.5 horse serum, 1% P/S and 10% optiprep^{™}. In case of PBMC-cells: RPMI-1640 with 10% FBS, 1% P/S and 10% Optiprep^{™}) was loaded into the sample inlet of a DE50 cartridge. The tri-block fluorosurfactant was dissolved in NovecTM 7500 Engineered fluid (3M^{™} A/S, Copenhagen S) to a concentration of 5% w/w, and the fluorosurfactant-solution (oil) was loaded into the oil inlet well of the cartridge. See example 9 for media and buffer components.

Cell culture medium and stabilizing solution for cells (Samplix, Birkerød, Denmark, cat # REDIVSTABSOL1500) were loaded into the outer medium inlet of the cartridge and double emulsion droplets were produced by inserting the cartridge into an Xdrop instrument (cat. no. IN00100, Samplix ApS, Birkerød, Denmark, fig. 13) and starting droplet production while following the recommendations of the manufacturer.

Microscope pictures were taken at 20x magnification within one hour of droplet production.

The droplets were left at room temperature for six weeks and the microscopy of the droplet productions was repeated.

The microscope pictures showed that less than 5% of the drops viewed in microscope were coalescent. Even after six weeks storage at room temperature and approximately 1 atmosphere of pressure less than 5% of the drops viewed in microscope were coalescent. Droplet coalescence was defined as a droplet having a diameter of at least 2 times the diameter of the average diameter of the observed droplets.

### Example 12: IR and NMR spectra of the fluorosurfactants.

### FTIR

Fourier transform infrared (FTIR) spectroscopy was carried out using a Nicolet iS50 FTIR spectrometer (Thermo Fisher Scientific, USA). The wavenumber between 400 and 4000 cm-1 was recorded for each sample in attenuated total reflection (ATR) mode.

The tri-block fluorosurfactant (82.5 % w/w in Novec 7500), the di-block fluorosurfactant ((96 % w/w in Novec 7500), and the Novec 7500 solvent (3M^{™} A/S, Copenhagen S) was analysed using ATR.

Figure 19 show a close-up of the most informative region between 3600 cm-1 and 1400 cm-1. Peak values are indicated. Also the background signal of the instrument is shown, and show that peaks between 2000 cm-1 and 2500 cm-1 mostly are due to background.

Strong electron withdrawing groups next to a carbonyl, results in a significant up-shift (https://www2.chemistry.msu.edu/faculty/reusch/virttxtjml/spectrpy/infrared/irspec1. htm). Accordingly, the peak at ca 1724 cm-1 is assigned the amide carbonyl stretch of the amide group. The peak at ca 2870 cm-1 and ca. 1450 cm-1 are probably associated with CH2 stretching and bending, and the ca 1538 cm-1 may be associated with NH bend in the amides.

The signal around 2300 cm-1 to 2400 cm-1 which also is seen in the background spectrum is probably an artifact caused by an imperfect ATR cell surface.

### NMR

¹⁹F Nuclear Magnetic Resonance was carried out using a Magritek SPINSOLVE 80 MHz Multi-X ULTRA. Novec 7500, tri-block fluorosurfactant (5 % w/w in Novec 7500) was analysed.

As expected from being a 5 % w/w the Novec 7500 solvent dominates the spectrum of the composition. However, the ¹⁹F Subtraction spectrum (Novec 7500 subtracted from the triblock signal), figure 20, did reveal triblock-specific signals, figure X2. The most dominant signal around -116 ppm most likely originates from the Fluorine in the repeating unit (c). The weak signal around -132 ppm most likely correspond to the fluorine next to the amide.

### CONCLUSION

The analysis indicates that the fluorosurfactants comprise an amide block as suggested by the synthesis scheme.

### Example 13: Bio-compatibility of the di- or the tri-block fluorosurfactant.

### Introduction

In this example it is shown that mammalian cells can grow in growth media with both the di- and the tri-block fluorosurfactants. Both the adherent cells HEK cells (Novo Nordisk, Bagsværd, Denmark) and the suspension cells Ramos cells (cat. no. CRL-1596, ATCC, Manassas, VA, USA) are used.

### Method

200,000 HEK cells were mixed with 1.34 ml media consisting of DMEM (cat. no. 10564011, Thermo Fisher Scientific, Waltham, MA, USA) with 10 % fetal bovine serum (cat. no. F9665, Sigma-Aldrich, Darmstadt, Germany) and 1 % penicillin streptomycin (cat. no. 15140122, Life Technologies, Waltham, MA, USA) and added to a well in a 6 well plate. 0.74 ml oil (5% w/w surfactant in Novec 7500) of either the fluorosurfactant having the tri-block, A-X-B-X-A formula, or the fluorosurfactant having the di-block, A-X-B formula was added to each well, too.

500,000 Ramos cells were mixed with 670 µl media consisting of RPMI-1640 (cat. no. 42401-018, Thermo Fisher Scientific) with 20 % fetal bovine serum and 1 % penicillin streptomycin and added to a well in a 24 well plate. 370 µl oil (5% w/w surfactant in Novec 7500) of either the fluorosurfactant having the tri-block, A-X-B-X-A formula, or the fluorosurfactant having the di-block, A-X-B formula, was added to the media in each well. The cells were incubated at 37 °C and 5 % CO₂ for 24 hours. The HEK cells were harvested by removing the oil and media, wash each well with 1 ml dulbecco phosphate buffered saline (14190-094, Thermo Fisher Scientific), add 200 µl trypsin (0.25 %) (cat. no. 25200056, Thermo Fisher Scientific)) to each well, and release cells from the well bottom with a cell scraper. 1.5 ml media was added to each well so the cell content could be transferred to a 2 ml tube. The Ramos cells were harvested by transferring each well's cell suspension to a 1.5 ml tube and remove the oil. Media was removed from harvested HEK and Ramos cells, which were resuspended in 100 µl media.

Then cells were dyed with Calcein acetoxymethyl (Calcein AM) (4µg/ml) (cat. no. 65-0853-39, Thermo Fisher Scientific, Waltham, MA, USA) and propidium iodide (PI) (1 µg/ml) (cat no. BMS500PI, Thermo Fisher Scientific) and incubated for 15-30 minutes at 37 °C and 5 % CO2.

Number of living and dead cells were counted as the number of Calcein- and PI-stained cells, respectively, using the CytoSmart instrument (CytoSmart, Eindhoven, The Netherlands).

### Results

Viability of HEK cells were high (>94 % living cells) in all wells, i.e. the oils did not seem to affect the viability of HEK cells (compare "No oil" to the rest of the data in figure 21A). The viability of Ramos cells depended somewhat on the used oil (Figure 21B). Viability decreased slightly when cells were exposed to the tri-block and the deblock oil compared to not exposed to oil: from an average of 97 % living cells to an average of 89 and 84% living cells for Xdrop DE oil I and V oil, respectively.

### Conclusion

The results show that the viability of cells in suspension is only slightly affected by the di- and triblock oil. The adherent HEK cells were not affected by the addition of oil to their growth medium.

**Example 14:** Viability of cells encapsulated in double-emulsion droplets stabilised by the di- or the tri-block fluorosurfactant.

### Introduction

In this example it is shown that mammalian cells stay viable in water-in-oil-in-water droplets when the applied oil is Xdrop DE Oil I. Suspension cells Ramos cells or natural killer (NK) cells were used.

### Method

Cells were stained with Carboxyfluorescein (Carboxyfluorescein succinimidyl ester, CFSE), and propidium iodide (PI) before they were encapsulated in droplets.

Specifically, in case of Ramos cells (cat. no. CRL-1596, ATCC, Manassas, VA, USA), they were stained with 1 µM CFSE (cat. no. C34570, Thermo Fisher Scientific, Waltham, MA, USA) in RPMI-1640 (cat. no. 42401-018, Thermo Fisher Scientific) with 20 % fetal bovine serum (cat. no. F9665, Sigma-Aldrich, Darmstadt, Germany) and 1 % penicillin streptomycin (cat. no. 15140122, Life Technologies, Waltham, MA, USA).

In case of NK cells (NK-92 cat. no. ACC 488, DSMZ) cells were stained with 1 µM CFSE (cat. no. C34570, Thermo Fisher Scientific, Waltham, MA, USA) in alfa-MEM medium with 12.5 % fetal bovine serum (cat. no. F9665, Sigma-Aldrich, Darmstadt, Germany), 12.5 % horse serum (spørger Mette I morgen hvor vi har dette fra), 1 % penicillin streptomycin (cat. no. 15140122, Life Technologies, Waltham, MA, USA), and 0.005 % IL-2

100,000 cells (Ramos or NK) in media were added to each cartridge lane for encapsulation in double emulsion droplets essentially as described in Example 3 but using use of a xDrop DE50 cartridge (see example 9 and figure 12) and a Xdrop instrument, (cat. no. IN00100, Samplix ApS, Birkerød, Danmark) to obtain double-emulsion droplets with a diameter of approximately 50 µm.

The sample cells were in 85 % media, 10 % optiprep (cat.no. 07820, Stemcell Technologies, Vancouver, Canada), and 5 % propidium iodide (1 µg/ml) (cat no. BMS500PI, Thermo Fisher Scientific).

Outer buffer consisted of 33 % 1x DE stabilizing solution for cells (cat.no. REDIVSTABSOL1500, Samplix, Birkerød, Denmark), 10 % optiprep, and 57 % media. The used oil was 5% w/w tri-block fluorosurfactant in Novec 7500

The cells were incubated in the DE50 droplets at 37 °C and 5 % CO₂ for up to 22 hours. Total number of cells and number of dead cells were determined after 3 and 22 hours of incubation by counting the number of CFSE- and PI-stained cells, respectively, with the Accuri 6 flowcytometry instrument (Becton Dickinson, Franklin Lakes, NJ, USA).

### Results

As seen in figure 22A the percentage of living cells were high after both 3 hours (94 %) and 22 hours (89 %) of incubation.

Similar, but slightly lower, viabilities were obtained with the relatively sensitive NK cells in droplets stabilised with either and the tri-block and di-block, 77% and 81%, respectively, after 23 hours of incubation, figure 22B.

**Example 15:** Permeability of membranes of double-emulsion droplets stabilised by the tri-block fluorosurfactant.

### Introduction

This example shows the permeability of double-emulsion droplets to two fluorophores: fluorescein and resorufin.

### Method

Double-emulsion droplets were made essentially as described in Example 3. Inner solution was 1x dPCR mix (cat.no. REMIXDE0170, Samplix, Birkerød, Denmark) containing 50 µM resorufin (cat.no. 73144, Sigma-Aldrich, Sigma-Aldrich, Darmstadt, Germany) or 5 µM fluorescein (cat.no. 46960, Sigma-Aldrich, Darmstadt, Germany) and was matched with an outer solution containing 1x dPCR buffer (cat.no. REBUFDE1500, Samplix, Birkerød, Denmark) and 50 µM resorufin or 5 µM fluorescein, respectively. For droplet productions with resorufin, Xdrop DE Oil I, V oil, Droplet Oil (DE), or FluoSurf was used. For droplet productions with fluorescein, only Xdrop DE Oil I was used. After droplet production, the outer solution was exchanged with another solution containing 1x dPCR buffer without resorufin or fluorescein. Immediately after buffer exchange, the droplet productions were analysed with flowcytometry on an Accuri 6 Plus flowcytometer (Becton Dickinson, Franklin Lakes, NJ, USA). Moreover, each resorufin-containing droplet production was analysed with flowcytometry after 15 min, 30 min, 60 min, 120 min, and 240 min, and each fluorescein-containing droplet production was analysed after 20 min, 60 min, and 180 min. Resorufin signal intensity was recorded with a filter that registers wavelengths at 585 nm +/- 22 nm, and fluorescein signal intensity was recorded with a filter that registers wavelengths at 530 nm +/- 15 nm. Half-time for the resorufin fluorescence signal decay was calculated using the Half-Life Calculator at calculator.net (09 June 2022).

### Results

See figure 23A and B.

### Conclusion

The droplets stabilized with the tri-block fluorosurfactant was considerably more permeable to resorufin than to fluorescein.

### REFERENCE NUMBERS

Any relevant part of the above disclosure may be understood in view of the below list of references in combination with the disclosed drawings.
1 Sample supply well, well C.
2 Hydrophobic (oil) phase feed well, well B.
3 External aqueous phase supply well, well A.
4 Collection well, well D
5 Protrusion for holding the gasket
6 IL-2 activated natural killer (NK) cell
7 NK cell
8 K562 target cell
9 Producer cell (NK-92 cells or Human Peripheral Blood Mononuclear Cells (PBMCs))
10 Interleukin-binding, fluorophore labelled antibody
11 Antibody binding to both cell surface and an interleukine
12 interleukin (IFN y or TNF α)
13 Interleukin-producing cell
14 cell not producing interleukin
15 only interleukin-producing cells will fluoresce.
21 Drop-generating cartridge, e.g. a Xdrop DE50 cartrige.
22 insert
23 Chamber 1 of the Split sample well insert
24 Septum / septum between chambers 1 and 2
25 Chamber 2
26 Exterior wall of insert
27 Chamber opening 1
28 Chamber opening 2
29 Mixing chamber
41 Touch screen
42 Start button
43 Drawer mechanism
44 Drawer door
49 Cassette
50 Gasket

### ITEMS OF THE SPECIFICATION

1. Method of synthesis of a fluorosurfactant having the formula: A-X-B or A-X-B-X-A, wherein:
   each instance of A is independently F-[CF(CF₃)CF₂O]_{c}-CF(CF₃)CONH-, wherein c is greater than or equal to 30;
   X, is a covalent bond; and
   each instance of B is independently -[C₃H₆O]_{d}-C₂H₄O]ₑ-[C₃H₆O]_{f}-CH₂CH(CH₃)- or -[C₃H₆O]_{g}-[C₂H₄O]ₕ-CH₃, wherein a combination of d and f is an integer greater than or equal to 2, e is an integer greater than or equal to 7, each of g and h are integers greater than 0 and a combination of g and h is an integer greater than or equal to 7;
   comprising the synthesis step of reacting the carboxylic end group of a fluoropolymer having the structure F-[CF(CF₃)CF₂O]_{c}-CF(CF₃)COOH with oxalyl chloride in the presence of N,N-Dimethylformamide to obtain an activated fluoropolymer intermediate having the structure F-[CF(CF₃)CF₂O]_{c}-CF(CF₃)COCl,
   wherein the activated fluoropolymer intermediate is subsequently reacted with a mono- or di-functionalized amine polymer of ethylene oxide (EO) and propylene oxide (PO) in a separate step.
2. The method of synthesis according to item 1, wherein the mono-functionalized amine polymer of ethylene oxide (EO) and propylene oxide (PO) has the structure:

   NH₂-[C₃H₆O]_{g}-[C₂H₄O]ₕ-CH₃ .
3. The method of synthesis according to item 1, wherein the di-functionalized amine polymer of ethylene oxide (EO) and propylene oxide (PO) has the structure:

   NH₂-[C₃H₆O]_{d}-[C₂H₄O]ₑ-[C₃H₆O]_{f}-CH₂CH(CH₃)-NH₂ .
4. The method of synthesis according to item 1, wherein the mono-functionalized amine polymer of ethylene oxide (EO) and propylene oxide (PO) has the structure:

   NH₂-[CH(CH₃)CH₂O]_{g}-[CH₂CH₂O]ₕ-CH₃ .
5. The method of synthesis according to item 1, wherein the di-functionalized amine polymer of ethylene oxide (EO) and propylene oxide (PO) has the structure:

   NH₂-[CH(CH₃)CH₂O]_{d}-[CH₂CH₂O]ₑ-[CH(CH₃)CH₂O]rCH₂CH(CH₃)-NH₂ .
6. The method of synthesis according to any one of the preceding items,
   wherein c is an integer between 39 and 44, the combination of d and f is an integer between 3 and 7, e is an integer between 7 and 20, and the combination of g and h is an integer between 7 and 25.
7. The method of synthesis according to any one of items 1-6, comprising the steps of:
   a. dissolving a species of the fluoropolymer having the structure F-[CF(CF₃)CF₂O]_{c}-CF(CF₃)COOH, wherein c is greater than or equal to 30, in a hydrofluoroether solvent,
   b. activating the carboxylic end group of the dissolved species of fluoropolymer by reaction with oxalyl chloride in the presence of N,N-Dimethylformamide to form an activated intermediate of said species,
   c. removing unreacted oxalyl chloride by heating the reaction under reduced pressure,
   d. reacting the activated intermediate of the species of fluoropolymer dissolved in dry hydrofluoroether solvent, with said mono- or said di-functionalized amine polymer of ethylene oxide and propylene oxide dissolved in an anhydrous mixture of dichloromethane and triethylamine, wherein
      the reaction is performed under N₂ atmosphere,
   e. filtering the product of step (d) and concentrating the filtrate to provide an orange oil residue comprising the reaction products of step (d),
   f. dissolving the orange oil residue in a 2:1 mixture of hydrofluoroether solvent and MeOH, and performing one or more rounds of phase separation to provide a phase enriched for the fluorosurfactant produced in step (d),
   g. optionally filtering the phase enriched for the fluorosurfactant in step (f) and concentrating the filtrate,
   wherein the hydrofluoroether solvent is preferably HFE-7100 or HFE-7500.
8. A composition producible by the method according to any one of the preceding items, comprising a fluorosurfactant having the formula: A-X-B or A-X-B-X-A, wherein:
   each instance of A is independently F-[CF(CF₃)CF₂O]_{c}-CF(CF₃)CONH-, wherein c is greater than or equal to 30;
   X, is a covalent bond; and
   each instance of B is independently -[C₃H₆O]_{d}-C₂H₄O]ₑ-[C₃H₆O]_{f}-CH₂CH(CH₃)- or -[C₃H₆O]_{g}-[C₂H₄O]ₕ-CH₃,
   wherein a combination of d and f is an integer greater than or equal to 2, e is an integer greater than or equal to 7, and wherein each of g and h are integers greater than 0 and a combination of g and h is greater than or equal to 7,
   wherein the composition comprising the fluorosurfactant having the formula: AX-B or A-X-B-X-A has single-emulsion stabilization properties characterized by the formation of an emulsion comprising said composition, wherein less than 5% of the single-emulsion drops formed are coalescent droplets after at least 16 hours incubation at 30°C and 10 min at 65 °C at about 1 atmosphere, wherein
   the coalescent droplets have an average cross-sectional dimension at least 2 times the average cross-sectional dimension of the observed droplets.
9. A composition producible by the method according to any one of items 1 - 7, comprising:
   a fluorosurfactant having the formula: A-X-B-X-A, wherein:
   each instance of A is independently F-[CF(CF₃)CF₂O]_{c}-CF(CF₃)CONH-, wherein c is greater than or equal to 30;
   X, is a covalent bond; and
   B is -[C₃H₆O]_{d}-[C₂H₄O]ₑ-[C₃H₆O]_{f}-CH₂CH(CH₃)-,
   wherein a combination of d and f is an integer greater than or equal to 2, e is an integer greater than or equal to 7, wherein the composition comprising the fluorosurfactant having the formula: A-X-B-X-A has double-emulsion stabilization properties characterized by the formation of an emulsion comprising said composition, wherein less than 5% of the single-emulsion drops formed are coalescent droplets after six weeks of storage at room temperature and
   approximately 1 atmosphere of pressure, when droplet coalescence is defined as a droplet having a diameter of at least 2 times the diameter of the average diameter of the observed double-emulsion droplets.
10. The composition according to item 8, wherein:

   A-X-B is F-[CF(CF₃)CF₂O]_{c}-CF(CF₃)CONH-[CH(CH₃)CH₂O]_{g}-[CH₂CH₂O]ₕ-CH₃

   and,

   A-X-B-X-A is F-[CF(CF₃)CF₂O]_{c}-CF(CF₃)CONH-[CH(CH₃)CH₂O]_{d}-[CH₂CH₂O]ₑ-[CH(CH₃)CH₂O]_{f}-CH₂CH(CH₃)NHCOCF(CF₃)-[OCF₂CF(CF₃)_{c}-F.
11. The composition according to items 8 - 10, wherein c is an integer between 39 and 44, the combination of d and f is an integer between 3 and 7, e is an integer between 7 and 20, and the combination of g and h is an integer between 7 and 25.
12. The composition according to items 8 - 10, wherein c is an integer between 40 and 43, the combination of d and f is an integer between 5 and 6, e is an integer between 12 and 13, g is an integer between 2 and 3 and h is an integer between 18 and 20.
13. A mixed composition comprising a combination of the composition according to any one of items 8, 10, 11 or 12 wherein the fluorosurfactant has the formula:
   A-X-B; and the composition according to any one of items 8 - 12 wherein the fluorosurfactant has the formula: A-X-B-X-A.
14. The mixed composition according to item 13, wherein the fluorosurfactant having the formula: A-X-B and the fluorosurfactant having the formula: A-X-B-X-A are combined in a ratio of between 1:5 and 1:20.
15. An emulsion composed of:
   i. aqueous droplets dispersed in an oil continuous phase (single emulsion droplets) or
   ii. oil-encapsulated aqueous droplets dispersed in an aqueous continuous phase (double emulsion droplets),
   wherein said emulsion comprises the composition or mixed composition according to any one of items 8 - 14, and wherein the oil is a fluorocarbon.
16. The emulsion according to item 15, wherein the aqueous droplets comprise reagents for PCR or MDA amplification of a DNA molecule.
17. The emulsion according to item 15, wherein the aqueous droplets comprise one or more cells.
18. A kit for generation of an emulsion, comprising a composition or mixed composition according to any one of items 8 - 14 in a first container, at least one microfluidic cartridge for forming single- and/or double- emulsion droplets, and a fluorocarbon oil in second container.
19. A method for forming stable double-emulsion droplets that are selective permeable comprising: the formation of double-emulsion droplets of a water-in-fluorocarbon oil-in-water emulsion, wherein said fluorocarbon oil emulsion comprises the composition or mixed composition according to any one of items 8 - 14.
20. A method for assaying the functionality of one or more cells comprising: encapsulating the one or more cells in double-emulsion droplets of a water-in-fluorocarbon oil-in-water emulsion, wherein said fluorocarbon oil emulsion comprises the composition or mixed composition according to any one of items 8 - 14.
21. The method according to item 20, wherein the method assays the functionality of one cell-type (the effector cell) acting upon another type (the target cell) comprising the steps:
   1) differential staining of the two cell-types,
   2) encapsulate the two cell types in plural double-emulsion droplets forming an double-emulsion, incubate the emulsion of double-emulsion droplets, and
   3) perform an analysis of the droplets by flowcytometry or imaging.
22. The method according to item 20 or 21, wherein the method assay the functionality of one cell-type (the effector cell) acting upon another type (the target cell) comprising the steps:
   1) preactivation of effector cells,
   2) differential staining of the two cell-types,
   3) washing and resuspending cells in a suitable medium,
   4) activate effector cells,
   5) encapsulate the two cell types in plural double-emulsion droplets forming an double-emulsion, incubate the emulsion of double-emulsion droplets, and
   6) perform an analysis of the droplets by flowcytometry or imaging.
23. The method according to item 20 or 21, wherein the effector cell is a CAR-T modified cell and the target cell is a cancer cell.
24. The method according to item 20, wherein the method assay the functionality of a population of cells for excretion of a specific substance comprising the steps:
   1) suspend the cells with an assay-composition which comprise:
      a. A reagent that binds to the surface of the cells and to the specific substance, and
      b. An antibody that binds to the same specific substance and which is coupled to a fluorophore.
   2) produce double-emulsion droplets containing cells and assay-components
   3) incubate double-emulsion droplets in a CO₂ incubator
   4) break the droplets to release the cells, and
   5) analyse the released cells by flowcytometry or imaging.
25. The method according to item 24, wherein the specific substance is a specific interleukin.
26. A method for obtaining a population of cells excreting a specific substance comprising:
   1) assaying a population of cells for excreting a specific substance using a FACS-instrument according to the method of item 24 or 25 while setting the FACS-instrument to collect the population of cells excreting the specific substance, and
   2) collect the population of cells excreting the specific substance.

## Claims

1. An emulsion composed of:
oil-encapsulated aqueous droplets dispersed in an aqueous continuous phase (double emulsion droplets),
wherein said emulsion comprises a composition comprising a fluorosurfactant having the formula: A-X-B or A-X-B-X-A, wherein:
each instance of A is independently F-[CF(CF₃)CF₂O]_{c}-CF(CF₃)CONH-, wherein
c is greater than or equal to 30;
X, is a covalent bond; and
each instance of B is independently -[C₃HₑO]_{d}-[C₂H₄O]ₑ-[C₃HₑO]_{f}-CH₂CH(CH₃)- or -[C₃H₆O]_{g}-[C₂H₄O]ₕ-CH₃,
wherein a combination of d and f is an integer greater than or equal to 2, e is an integer greater than or equal to 7, and wherein each of g and h are integers greater than 0 and a combination of g and h is greater than or equal to 7,
wherein the composition comprising the fluorosurfactant having the formula: A-X-B or A-X-B-X-A has double-emulsion stabilization properties **characterized by** less than 5% of the drops viewed in microscope are coalescent, after the emulsion has been subjected to thermocycling comprising at least 40 repetitive incubations at 94°C for 3 sec followed by 60°C for 30 sec t approx. 1 atmosphere, wherein the coalescent droplets have an average cross-sectional dimension at least 2 times the average cross-sectional dimension of the droplets formed, and wherein the size and number of the single-emulsion droplets and the coalesced droplets is determined by bright-field microscopy,
wherein the oil is a fluorocarbon, and wherein the aqueous droplets comprise one or more cells.

2. An emulsion composed of:
oil-encapsulated aqueous droplets dispersed in an aqueous continuous phase (double emulsion droplets),
wherein said emulsion comprises a composition comprising a fluorosurfactant having the formula: A-X-B-X-A, wherein:
each instance of A is independently F-[CF(CF₃)CF₂O]_{c}-CF(CF₃)CONH-, wherein
c is greater than or equal to 30;
X, is a covalent bond; and
B is -[C₃H₆O]_{d}-[C₂H₄O]ₑ-[C₃H₆O]_{f}-CH₂CH(CH₃)-,
wherein a combination of d and f is an integer greater than or equal to 2, e is an integer greater than or equal to 7,
wherein the composition comprising the fluorosurfactant having the formula: A-X-B-X-A has double-emulsion stabilization properties **characterized by** the formation of an emulsion comprising said composition, wherein less than 5% of the single-emulsion drops formed are coalescent droplets after six weeks of storage at room temperature and approximately 1 atmosphere of pressure, when droplet coalescence is defined as a droplet having a diameter of at least 2 times the diameter of the average diameter of the observed double-emulsion droplets
wherein the oil is a fluorocarbon, and wherein the aqueous droplets comprise one or more cells.

3. The emulsion according to claim 1 or 2, wherein the composition is a mixed composition comprising a combination of the composition according to claim 1 wherein the fluorosurfactant has the formula: A-X-B; and the composition according to claim 2 wherein the fluorosurfactant has the formula: A-X-B-X-A.

4. The emulsion of claim 1 or 3 comprising a di-block fluorosurfactant A-X-B whose chemical structure is:

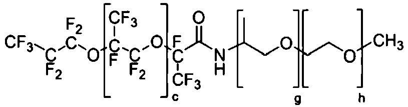

5. The emulsion according to any one of the claims 1-4 comprising a tri-block fluorosurfactant A-X-B-X-A whose chemical structure is:

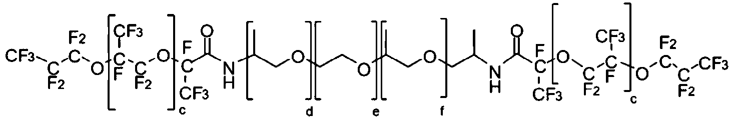

6. The emulsion according to claim 4 or 5, wherein c is an integer between 30 and 50, the combination of d and f is an integer between 3 and 7, e is an integer between 7 and 20, and the combination of g and h is an integer between 7 and 25.

7. A method for forming stable double-emulsion droplets that are selective permeable comprising: the formation of double-emulsion droplets of a water-in-fluorocarbon oil-in-water emulsion, wherein said emulsion comprises a composition or mixed composition
wherein said emulsion is as defined according to any one of the claims 1-6.

8. A method for assaying the functionality of one or more cells comprising
encapsulating the one or more cells in double-emulsion droplets of a water-in-fluorocarbon oil-in-water emulsion, wherein anemulsion according to any one of claims 1-6 is obtained.

9. The method according to claim 8, wherein the method assays the functionality of one cell-type (the effector cell) acting upon another type (the target cell) comprising the steps:
1) differential staining of the two cell-types,
2) encapsulate the two cell types in plural double-emulsion droplets forming an double-emulsion, incubate the emulsion of double-emulsion droplets, and
3) perform an analysis of the droplets by flowcytometry or imaging.

10. The method according to claim 8 or 9, wherein the method assay the functionality of one cell-type (the effector cell) acting upon another type (the target cell) comprising the steps:
1) preactivation of effector cells,
2) differential staining of the two cell-types,
3) washing and resuspending cells in a suitable medium,
4) activate effector cells,
5) encapsulate the two cell types in plural double-emulsion droplets forming an double-emulsion, incubate the emulsion of double-emulsion droplets, and
6) perform an analysis of the droplets by flowcytometry or imaging.

11. The method according to any one of claims 8-10, wherein the effector cell is a CAR-T modified cell and the target cell is a cancer cell.

12. The method according to any one of claims 8-11, wherein the method assays the functionality of a population of cells for excretion of a specific substance comprising the steps:
1) suspend the cells with an assay-composition which comprise:
a. A reagent that binds to the surface of the cells and to the specific substance, and
b. An antibody that binds to the same specific substance and which is coupled to a fluorophore.
2) produce double-emulsion droplets containing cells and assay-components
3) incubate double-emulsion droplets in a CO₂ incubator
4) break the droplets to release the cells, and
5) analyse the released cells by flowcytometry or imaging.

13. The method according to claim 12, wherein the specific substance is a specific interleukin.

14. A method for obtaining a population of cells excreting a specific substance comprising:
1) assaying a population of cells for excreting a specific substance using a FACS-instrument according to the method of claim 12 or 13 while setting the FACS-instrument to collect the population of cells excreting the specific substance, and
2) collect the population of cells excreting the specific substance.

15. The population or strain of cells obtained by the method of claim 14.
